# EUROPEAN PATENT SPECIFICATION

(11) **EP 0 758 397 B1**
(45) Date of publication and mention of the grant of the patent: **22.06.2005**
(21) Application number: 95919361.6
(22) Date of filing: 28.04.1995
(51) Int. Cl.: C12N 15/86, C12N 7/01, C12N 15/63, A01K 67/00, C12N 15/16, A61K 39/00

(54) **RECOMBINANT POXVIRUSES WITH FOREIGN POLYNUCLEOTIDES IN ESSENTIAL REGIONS**
REKOMBINANTE POXVIREN MIT FREMDENPOLYNUKLEOTIDEN IN WICHTIGEN REGIONEN
POXVIRUS RECOMBINES DANS DES REGIONS ESSENTIELLES A L'AIDE DE POLYNUCLEOTIDES ETRANGERS

(30) Priority: 29.04.1994 US 235392
(43) Date of publication of application: 19.02.1997
(73) Proprietor: Baxter Healthcare S.A., 8304 Wallisellen (CH)
(72) Inventor: FALKNER, Falko-Günter, 2304 Orth/Donau (AT); HOLZER, Georg, 1060 Wien (AT); DORNER, Friedrich, A-1230 Wien (AT)
(74) Representative: Polz, Leo, Dr.
(86) International application number: PCT/EP1995/001629
(87) International publication number: WO 1995/030018

(56) References cited:
- US-A- 5 225 336
- JOURNAL OF VIROLOGY, vol. 63, no. 9, 1989 pages 3829-3836, M.E. PERKUS ET AL. 'Cloning and expression of foreign genes in vaccinia virus using a host range selection system'
- JOURNAL OF VIROLOGY, vol. 64, no. 6, 1990 pages 3108-3111, F.G. FALKNER ET AL. 'Transient dominant selection of recombinant vaccinia viruses'
- HOLZER G.W.; FALKNER F.G.: 'Construction of a vaccinia virus deficient in the essential DNA repair enzyme uracil DNA glycosylase by a complementing cell line', J. Virol. 1997, vol 71, no 7, p 4997-5002
- HIMLY ET AL: 'Defective vaccinia virus as a biologically safe tool for the overproduction of recombinant human secretory proteins', Prot. Expr. and Purif., 1998, vol 14, p 317-26

### SEQUENCE LISTING

(1) GENERAL INFORMATION:
   (i) APPLICANT:
      (A) NAME: IMMUNO AG
      (B) STREET: Industriestr. 67
      (C) CITY: Wien
      (E) COUNTRY: AUSTRIA
      (F) POSTAL CODE (ZIP): A-1221
   (ii) TITLE OF INVENTION: Recombinant poxviruses with foreign polynucleotides in essential regions
   (iii) NUMBER OF SEQUENCES: 40
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: US 08/235,392
      (B) FILING DATE: 29-APR-1994
(2) INFORMATION FOR SEQ ID NO: 1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 1:
   AGTACTCCAT GGAAAGATAT ACAGATTTAG 30
(2) INFORMATION FOR SEQ ID NO: 2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 2:
   ATCCCGGGTT TTAGAGACTT TGAAGCTACT 30
(2) INFORMATION FOR SEQ ID NO: 3:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 3:
   CTAGGCTTTT GCAAAAAGCT CCTCGACCAT GGTGTCTTCA 40
(2) INFORMATION FOR SEQ ID NO: 4:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 4:
   AGCTTGAAGA CACCATGGTC GAGGAGCTTT TTGCAAAAGC 40
(2) INFORMATION FOR SEQ ID NO: 5:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 5:
   AGGAGTTAAT GAGGCCAATG GA 22
(2) INFORMATION FOR SEQ ID NO: 6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 6:
   GACATAGGTA TAGAATCCGG A 21
(2) INFORMATION FOR SEQ ID NO: 7:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 7:
   AGTTTAAACG GCGCGCCCGG GCTCGAGAGG CCTCTGCAGA TGCATCCATG GGGATCCGAA 60
   TTC 63
(2) INFORMATION FOR SEQ ID NO: 8:
   (i) SEQUENCE CHARACTERISTICS:
      (A) LENGTH: 63 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 8:
   GAATTCGGAT CCCCATGGAT GCATCTGCAG AGGCCTCTCG AGCCCGGGCG CGCCGTTTAA 60
   ACT 63
(2) INFORMATION FOR SEQ ID NO: 9:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 9:
   GATCCTACGT ATCTAGAATT AATTAATGGC CAATTTAAAT GCCCGGGA 48
(2) INFORMATION FOR SEQ ID NO: 10:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 10:
   AATTTCCCGG GCATTTAAAT TGGCCATTAA TTAATTCTAG ATACGTAG 48
(2) INFORMATION FOR SEQ ID NO: 11:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 11:
   CTCGTAAAAA TTGAAAAACT ATTCTAATTT ATTGCACGGT CGCGA 45
(2) INFORMATION FOR SEQ ID NO: 12:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 12:
   CATGGTACGT ACCGTGCAAT AAATTAGAAT AGTTTTTCAA TTTTTACGAG 50
(2) INFORMATION FOR SEQ ID NO: 13:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 13:
   AAACTGCAGG ATGCGATATA TAGTAAGTCC GCAAATGGTA TTA 43
(2) INFORMATION FOR SEQ ID NO: 14:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 14:
   AGGAATTCAG GCCTGTTTAA TTAATTTGTG CTCTTC 36
(2) INFORMATION FOR SEQ ID NO: 15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 15:
   AGCGCCGCTA CTAGCACTCC 20
(2) INFORMATION FOR SEQ ID NO: 16:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 16:
   GAACGTTAAC ATTTATATCG TGGGGTAAAG TGAAAATC 38
(2) INFORMATION FOR SEQ ID NO: 17:
   (1) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 17:
   TTGGAGAACT TGATACGCCG 20
(2) INFORMATION FOR SEQ ID NO: 18:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 18:
   CATATGCAAT TGTAAATTAA ACAACTAAAT CTGTAAATAA ATA 43
(2) INFORMATION FOR SEQ ID NO: 19:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 19:
   AACGGTCCGG CCCGGGCGGC CGCC 24
(2) INFORMATION FOR SEQ ID NO: 20:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 20:
   AATTGGCGGC CGCCCGGGCC GGACCGTT 28
(2) INFORMATION FOR SEQ ID NO: 21:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 21:
   CTGCAGAATG AATACCGGAA TTATAGATTT 30
(2) INFORMATION FOR SEQ ID NO: 22:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 22:
   CCCGGGTTAT GGAGAAGATA CCACGTT 27
(2) INFORMATION FOR SEQ ID NO: 23:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 23:
   TGAGAAGAAT TGCCGTCG 18
(2) INFORMATION FOR SEQ ID NO: 24:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 24:
   GTCGACGATA TCTTCTATAA ATATATGAGC 30
(2) INFORMATION FOR SEQ ID NO: 25:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 25:
   TACAGTACCC GAATCTCTAC 20
(2) INFORMATION FOR SEQ ID NO: 26:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 26:
   ACAATTGGTA TTAAGTATAA CGACTCC 27
(2) INFORMATION FOR SEQ ID NO: 27:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 27:
   GCCATGGATC ACAACCAGTA TCTCTT 26
(2) INFORMATION FOR SEQ ID NO: 28:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 28:
   ACCCGGGTTA ATTAATTATT GTTCACTTTA 30
(2) INFORMATION FOR SEQ ID NO: 29:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 29:
   TCCACATCCA TGAGATTGAA T 21
(2) INFORMATION FOR SEQ ID NO: 30:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 30:
   CGATTGATAC ATATCATTAC C 21
(2) INFORMATION FOR SEQ ID NO: 31:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 31:
   AACAATTGCA TCATCTGCGA AGAACATCG 29
(2) INFORMATION FOR SEQ ID NO: 32:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 32:
   CAGTTAACTT TTCAGGTAAA AGTACAGAAT 30
(2) INFORMATION FOR SEQ ID NO: 33:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 33:
   ACCATGGACT CTAAAGAGAC TAT 23
(2) INFORMATION FOR SEQ ID NO: 34:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 34:
   CAATTGCCCG GGTTAATTAA TGAAATTAAT CATATACAAC TC 42
(2) INFORMATION FOR SEQ ID NO: 35:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 35:
   CCTTTAGGTC AGAAGATCGC 20
(2) INFORMATION FOR SEQ ID NO: 36:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 36:
   GTCGACAATT GTTAAAGTAC AAACAACTAG GA 32
(2) INFORMATION FOR SEQ ID NO: 37:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 37:
   GCTAACATCA TACCCTCCTG 20
(2) INFORMATION FOR SEQ ID NO: 38:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 38:
   GTCGACGTTA ACAGAAGCAT TGGAATACGC AT 32
(2) INFORMATION FOR SEQ ID NO: 39:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 39:
   TACGTATGGA GACCAACACC CTTCC 25
(2) INFORMATION FOR SEQ ID NO: 40:
   (i) SEQUENCE CHARACTERISTICS:
      (A) LENGTH: 32 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 40:
   CCATGGATAT CGGGTTCCCT GCTCTCTGTC GG                                      32

## Description

Recombinant poxviruses have been used as vectors for expression of foreign genes from a variety of sources, such as animals, plants, protozoa, fungi, bacteria, and viruses. Additionally, recombinant poxviruses can be used to express chimeric genes and genes of synthetic origins. Typically, chimeric genes and synthetic genes are DNA sequences that are derived from or based on naturally-occurring sequences. For example, cDNA sequences generated from RNA transcripts, including those from retroviruses, can be expressed with recombinant poxvirus vectors.

Poxvirus vectors in present use contain foreign DNA inserted into genomic regions that are not essential to the viability of the virus. These poxviruses expressing foreign genes are usually constructed by insertion of the foreign sequences into genomic regions that are non-essential for growth of the virus in cell culture by homologous recombination. See Panicali and Paoletti, Proc. Nat'l Acad. Sci. USA 79: 4927-31 (1982); Mackett et al., Proc. Nat'l Acad. Sci. USA 79: 7415-19 (1982). Preferably, recombinant poxviruses can be constructed via direct molecular cloning. See EP 0 561 034; Scheiflinger et al., Proc. Nat'l Acad. Sci. USA, 89: 9977-81 (1992); Merchlinsky and Moss, Virol. 190: 522-26 (1992). Viruses with foreign DNA inserted into non-essential regions can grow autonomously in their host organism and, thus, remain infectious to their respective hosts.

The absence of the products of non-essential regions does not unduly impair the viability of the recombinant poxvirus. In the case of vaccinia, often considered the prototypic poxvirus, one of the major non-essential regions for insertion is the thymidine kinase gene. When a foreign gene is inserted into the vaccinia thymidine kinase gene, the product of the gene is lost to the virus. Nevertheless, the recombinant vaccinia is still viable. Other poxviruses also are amenable to similar manipulations in other non-essential regions. For example, recombinant avipox, such as fowlpox, have been developed where foreign DNA has been inserted into non-essential regions.

Recombinant vaccinia can be used as live vaccines, in large-scale expression systems, and a variety of other purposes. See Miner & Hruby, TIBTECH 8: 20-25 (1990). The use of these recombinant viruses for large-scale expression of proteins presents significant risks, however, because the viruses retain an infective capability. Accordingly, costly precautions and cumbersome procedures are required when handling recombinant vaccinia. For example, thermal inactivation of all media, fluids and contaminated lab ware is required, as well as special training for all personnel handling the virus. Several approaches have been considered for minimizing the need for these elaborate precautions. For example, highly attenuated vaccinia strains have been developed. These highly attenuated strains have limited infectivities. Accordingly, production of proteins with these highly attenuated strains has been attempted. Unfortunately, highly attenuated virus strains have slow and limited growth, which often makes these strains poorly suited for large-scale protein production.

In addition to the highly attenuated poxviruses, impaired strains of other viruses are known. For example, impaired retroviruses can be grown in helper cell lines that are capable of complementing the impaired virus. See Kriegler, GENE TRANSFER AND EXPRESSION (Stockton Press, 1990) at 33-39; Mann et al., Cell 33: 153-59 (1983). Retroviruses, however, are small RNA containing viruses that multiply by integration into the genome of the host cell, which makes these viruses inappropriate for use as expression vectors or vaccines.

Impaired adenoviruses grown in complementing cells have been used as vaccines, as described by Eloit et al., J. Gen. Virol. 71: 2425-31 (1990). Adenoviruses are double-stranded DNA viruses that, in contrast to vaccinia virus, replicate in the nucleus of infected cells and have a narrow host range. Additionally, the growth of impaired herpes virus in helper cells has also been reported. See PCT publications WO 94/03595, WO 94/21807 and WO 92/05263. Herpes virus also replicates in the nucleus of infected cells. Naked herpes DNA, like adenovirus DNA, is infectious. Accordingly, the promoters of these viruses function normally in the host cell.

In contrast to poxviruses, herpes and adenoviruses are nuclear viruses. Defective viruses, however, derived from nuclear viruses have the potential of rescuing defective regions from the host cell via homologous recombination. Such recombinational events are undesirable and dangerous because the impaired virus can potentially return to a wild-type state, which results in increased infectivity and virulence, as well as in the loss of the inserted foreign gene.

Because of the need for safer recombinant expression systems and vaccines, inventive approaches are required for engineering fundamentally different recombinant poxviruses. The approach described here entails insertion of a foreign gene into an essential region of the poxvirus.

It thus is an object of the present invention to provide recombinant poxviruses capable of expressing foreign polynucleotides, such as genomic DNA and cDNA.

It is another object of the present invention to provide recombinant poxviruses that are defective because they lack a function imparted by an essential region of the poxvirus genome.

It is another object of the present invention to provide defective poxviruses that possess foreign polynucleotides inserted into essential regions of the poxvirus genome.

It is still another object of the present invention to provide defective poxviruses that have all or part of an essential region deleted.

It is still another object of the present invention to provide defective poxviruses that can be used as vaccines.

In accomplishing these and other objects of the present invention, there are provided, in accordance with one aspect of the invention, defective poxviruses that lack a function imparted by an essential region of its parental poxvirus, the defective poxvirus comprising a foreign polynucleotide under transcriptional control of a promoter. That is, the defective poxvirus is derived from a parental poxvirus, but lacks a function that is normally present in the parental poxvirus.

The promoter should be operable with the enzymes of the defective poxvirus or the infected host. Such promoters include poxvirus promoters and synthetic promoters based upon poxvirus promoters. The foreign polynucleotide can be inserted into the essential region or replace some or all of the essential region.

Additionally, a marker can substitute for the essential region prior to insertion of the foreign polynucleotide into the virus. The foreign polynucleotide can be then inserted into the marker or replace the marker. Preferably, the parental poxvirus is vaccinia and the essential functions to be lost are encoded by open reading frames I7L, F18R, F13L, D13L, D6R, A8L, H4L, J1R, J3R, A10L and A3L or open reading frames that encode a 14 kilodalton envelope protein or a 25 kilodalton structural protein.

In accordance with still another aspect of the present invention, there is provided a method of producing a protein, comprising the step of providing a defective poxvirus that lacks a function imparted by an essential region of its parental poxvirus, wherein the defective poxvirus comprises a foreign polynucleotide encoding the protein to be produced, and wherein the polynucleotide is under transcriptional control of a promoter. The promoter should be operable with the defective poxvirus or infected host, such promoters include poxvirus promoters. The polynucleotide can be inserted into the essential region of the parental poxvirus. In another embodiment, a marker, such as the gpt gene, can replace some or all of the essential region and, in turn, the polynucleotide can be inserted into the marker or replace some or all of the marker. Loss of the marker function then will indicate that the polynucleotide has been placed within the defective poxvirus.

In accordance with yet another aspect of the present invention, there are provided vaccines comprising defective poxviruses. These defective poxviruses comprise polynucleotides that encode antigens against pathogens. The poxviruses for vaccines of the present invention can be constructed in the same or similar manner as the other defective poxviruses of the present invention.

These and other aspects of the invention disclosed herein will become apparent to the skilled artisan in view of the disclosure contained herein.
FIGURE 1 schematically depicts the construction of plasmid pRSET-I7L.
FIGURE 2 schematically depicts the construction of plasmid pSV-I7L-EDH.
FIGURE 3 schematically depicts the construction of plasmid pCR-I7Lf-ZG.
FIGURE 4 schematically depicts the construction of plasmid pRSET-A8L-3'/2
FIGURE 5 schematically depicts the construction of plasmid pSV-A8L-EDH.
FIGURE 6 schematically depicts the construction of plasmid pCR-A8Lf-ZG.
FIGURE 7 schematically depicts the construction of plasmids pRSET-D6R and pRSET-D6R-3'.
FIGURE 8 schematically depicts the construction of plasmid pSV-D6R-EDH.
FIGURE 9 schematically depicts the construction of plasmid pCR-D6Rf-ZG.
FIGURE 10 schematically depicts the construction of plasmid pRSET-J1R.
FIGURE 11 schematically depicts the construction of plasmid pSV-J1R-EDH.
FIGURE 12 schematically depicts the construction of plasmid pCR-J1R-ZG.
FIGURE 13 schematically depicts the construction of plasmid pSV-H4L-EDH.
FIGURE 14 schematically depicts the construction of plasmid pCR-H4Lf-ZG.
FIGURE 15 schematically depicts the construction of plasmid pHS-I7L.

The present invention relates in one aspect to the use defective poxviruses for the production of proteins. A "defective poxvirus" according to the present invention is a poxvirus that lacks a function imparted by an essential region of a parental poxvirus upon which the defective poxvirus is based; as a consequence, the defective poxvirus is not viable or infective without complementation of the lost function by another source. An "essential region" is a region of the poxvirus genome that is needed for viability or infectivity of the parental poxvirus. The term "complementation" connotes a restoration of a lost function by another source, such as a host cell. Thus, a defective poxvirus is a non-viable or non-infective form of a parental poxvirus, and can become viable or infective in the presence of complementation.

A parental poxvirus can be a naturally-occurring poxvirus or poxvirus derived therefrom.

A defective virus is based upon (derived from) parental poxviruses such as naturally-occurring poxviruses or poxviruses derived from naturally-occurring poxviruses. These derived poxviruses for use as parental poxviruses can be obtained by techniques such as genetic engineering via region-specific and site-specific mutagenesis, in vivo recombination, passaging of virus through host cells in the presence or absence of mutagens, and any combination of the above. A defective poxvirus can be based upon any type of poxvirus in which a defect in an essential region can be introduced.

The defective poxvirus can be used as an expression system for proteins. The proteins produced by the defective poxviruses of the present invention are encoded by foreign polynucleotides, such as genes or cDNA versions of RNA transcripts. The term "foreign polynucleotide" refers to a polynucleotide that is not normally present in the parental poxvirus upon which the defective poxvirus is based, or is a polynucleotide that is present in a different location or form than would be found in the parental poxvirus upon which the defective poxvirus is based. The foreign polynucleotides can be from a variety of sources, such as animals, plants, protozoa, fungi, bacteria, and viruses.

A defective poxviruses of the present invention can include a promoter that is operable with the defective poxvirus or host infected by the defective poxvirus in order to control the transcription of the foreign polynucleotide within the defective poxvirus. A "promoter" refers to a nucleotide sequence which can initiate and/or direct transcription. Any promoter that is operable with the defective poxvirus or within the infected host can be used in the present invention. "Operability" denotes recognizability or readability of the prompter by transcriptional enzymes. Typically, the promoters are poxvirus promoters or synthetic promoters based on promoters of poxvirus origin.

A defective virus can have at least one foreign polynucleotide inserted into an essential region of the poxvirus. A promoter can be linked to the foreign polynucleotide so that the promoter is inserted along with the polynucleotide in order for the promoter to have transcriptional control over the polynucleotide. In the alternative, the promoter for controlling transcription of the foreign polynucleotide can be already present in the defective poxvirus in a location that will allow the promoter to control transcription of the foreign polynucleotide.

The essential region may be fully deleted from the genome of the defective virus so that no homologous recombination is possible between the genome of the complementation source and the defective virus. A defective virus with such a deletion can only grow in the presence of the complementation source, such as a helper cell line that provides the function of the essential region in trans. Pure stocks of the defective virus can be grown in the helper cell line while growth and multiplication in normal cells and organisms remain impossible. The host range of this type of virus can be restricted to helper cell lines that are specifically engineered to complement the defect of the defective virus. A marker gene can be used to replace an essential region in the defective poxvirus. The foreign polynucleotide can then be inserted into the marker or replace all or part of the marker by homologous recombination. This approach provides a selection system for poxviruses containing inserts of interest.

The present invention is useful for more than large scale expression of proteins. The defective poxviruses of the invention also can be used for vaccination purposes. For example, if an appropriate essential gene is inactivated, the defective viruses still retain their ability to penetrate into cells and start an abortive life cycle. Such a gene would be one that is required late in the life cycle and, therefore, the virus still able to replicate its DNA, eventually form immature particles and express its genomic information, which includes the foreign polynucleotide. In this respect, these defective viruses could have the effect of the "dead live vaccines." Taylor and Paoletti, Vaccine 6: 466-68 (1988). A defective poxvirus that is used as a vaccine would comprise a DNA polynucleotide that encodes an antigen. Antigens are molecules that an organism recognizes as being foreign. These antigens can induce an immunological response from the organism exposed to the antigen. Antigens include proteins of bacterial, viral, fungal and protozoan origin. A preferred parental vaccinia strain for the construction of defective viruses to be used as vaccines would be vaccine strains such as the New York City Department of Health Laboratories strain (ATCC VR-325).

The defective poxviruses of the present invention can be grown in host cells that can complement the lost essential function of the defective virus. Typically, these host cells are from cell lines that are specifically engineered to complement the lost function. Typically, the host cell will have the same or similar poxvirus essential region inserted in the host cell in such a manner that the essential region is expressed by the host cell or the essential region in the cell is inducible upon infection of the host cell.

Essential gene products suitable as complementing agents include, in general, gene products that are required late in the poxvirus life cycle. Illustrative of such gene products are those involved in morphogenesis or other post-replication events, as well as enzymes and regulatory proteins.

One essential gene product that is a suitable complementing agent is the 47-kDa protein (the "17 protein") encoded by open reading frame ("orf") I7L, which is believed to be involved in viral genome organization. Kane and Shuman, J. Virol.67: 2689-98 (1993). The 17 protein is encapsulated with the virus core. A temperature sensitive (ts) mutation exists known as ts16. Condit et al., Virol. 128: 429-43 (1983). The I7 gene therefore fulfills a classic requirement of an essential gene, which is the existence of a conditional lethal mutant (temperature sensitivity).

Among the essential gene products that are suitable complementing agents in the present context is the 65kDa protein encoded by the D13L orf. This protein is expressed late in infection. If expression is prevented replication is unaffected while viral morphogenesis is blocked at an early stage. See Zhang and Moss, Virol. 187: 643-53 (1992). Other essential gene products that are suited as complementing agents are the vaccinia 14 kDa envelope protein, Lai et al., J. Biol. Chem. 265: 22174-80 (1990); the vaccinia 25kDa major structural protein, Weir and Moss, J. Virol. 56: 534-40 (1985); and other structural proteins such as P4a and P4b.

Another essential gene product that can be used as a complementing agent is the 11kDa phosphoprotein, Wittek et al., J. Virol. 49: 371-78 (1984), encoded by the F18R orf of WR wild type virus (known as "F17R" of the Copenhagen strain of vaccinia). See Goebel et al., Virol. 179: 247-66 (1990). The 11kDa phosphoprotein encoded by the F18R orf is an essential gene that is required for correct virion assembly. See Zhang and Moss, J. Virol. 65: 6101-10 (1991). Conditional lethal vaccinia mutants in the F18R and F17R orfs form immature particles with aberrant internal structures under certain conditions. F18R overlaps with the orf A, however, and thus generally would not be the essential region of first choice. Goebel et al., supra.

Enzymes and regulatory proteins also can be employed as complementing agents because these proteins need only be present in catalytic amounts, which can be readily provided by the complementing cell line. The subunits of the vaccinia virus early transcription factor ("VETF") are suitable in this respect. The VETF is essential for the initiation of transcription of "early" vaccinia genes. Vaccinia RNA polymerase lacking VETF is unable to transcribe double-stranded DNA templates in vitro. See Broyles et al., J. Biol. Chem. 263: 10754-60 (1988). The factor is a heterodimer having a 77kDa polypeptide and a 82kDa polypeptide, which are respectively encoded by the D6R and A8L gene orf of the vaccinia WR genome. See Gershon and Moss, Proc. Nat'l Acad. Sci. USA 87: 4401-05 (1990).

VETF proteins are expressed late in infection and packaged into the nascent virus particles. A defective virus lacking the D6R or the A8L gene isolated from VETF expressing cells should therefore be able to perform an additional complete life cycle on a non-complementing cell line without further limitations of protein synthesis. Protein synthesis is an essential requirement for the use of such defective viruses as "dead-live vaccines."

Dead-live vaccines include avipoxviruses expressing foreign genes that used to immunize mammalian hosts. In the mammalian host, the viruses do not multiply, but rather undergo an abortive life cycle. Nevertheless, this type of vaccine stimulates immune response of particular T cells. See Taylor and Paoletti, supra. A defective virus lacking VETF is desirable because it should be able to perform an additional life cycle in wild-type hosts as compared to viruses capable only of performing abortive life cycles, which allows for greater antigen production.

Another viral factor that is suitable for complementation is the RNA polymerase associated protein RAP 94 or early transcription specifity factor. RAP94 is encoded by the orf H4L, Ahn and Moss, Proc. Nat'1 Acad. Sci: USA 89: 3536-40 (1992), and it is a subunit of the viral DNA dependent RNA polymerase that can be disassociated. The subunit confers promotor specifity to the RNA polymerase complex for the initiation of transcription of early stage genes. As complementing agent it fulfills the same requirements as the VETF subunits. RAP 94 is synthesized late in infection and packaged into the nascent virus to mediate early transcription in the following round of infection. Hence, phenotypically intact virus particles lacking the H4L gene should be able to perform one additional round of infection in a non-complementing host without further limitations on protein synthesis. The existence of conditionally lethal mutants, Condit and Motytzca, Virol. 113: 224-41 (1981), confirms that H4L is an essential gene. The protein is found to be present in the virion in submolar amounts compared to other components of the RNA polymerase complex. Ahn and Moss supra. Accordingly, low expression levels of RAP94 in the complementing cell line should not affect growth of the defective virus.

Another gene that may be used for complementation of defective virus is the J1R gene, which is essential for virus growth in cell culture. Although its gene product has not been identified so far, this orf is of considerable interest due to its location adjacent to the thymidine kinase gene on the vaccinia virus genome. Deletion of a sequence that includes the orf J1R and at least parts of the adjacent thymidine kinase gene (J2R) results in a virus which is dependent on a complementing cell line and which is at the same time selectable by negative selection on tk-negative cells such as LM(TK-) cells or tk-negative Vero cells. Pure stocks of defective virus are rapidly obtained using this additional selection procedure.

The J3R gene is of interest in the same respect as the J1R orf. Negative selection on tk-negative cells can be performed due to the simultaneous deletion of the J3R orf and the adjacent J2R orf (thymidine kinase). The J3R gene encodes a poly(A)polymerase subunit (VP39) that is required for the formation of the 5' cap structure of mRNA and that stimulates the formation of long poly(A) tails. Gershon et al., Cell 66: 1269-78 (1991); Schnierle et al., Proc. Nat'l Acad. Sci. USA 89: 2897-01 (1991).

Defective poxviruses of the present invention can be produced with many of the same methods used to construct recombinant viruses having inserts in non-essential regions. For example, in vivo recombination via homologous flanking region can be used. See Panicali and Paoletti, Proc. Nat'l Acad. Sci. USA 79: 4927-31 (1982); Mackett et al., loc. cit. 79: 7415-19 (1982). An alternative method of producing defective poxviruses is direct molecular cloning and heterologous packaging. See EP 0 561 034; Scheiflinger et al., Proc. Nat'l Acad. Sci. USA 89: 9977-81 (1992); Merchlinsky and Moss, Virol. 190: 522-26 (1992).

### Example 1: Construction of a defective vaccinia virus lacking the I7L-protein ("d-I7L-ZG") and a complementing cell line based on Vero-cells ("V-I7L")

### Construction of Complementing Cell Line V-I7L

The first step in the construction of the helper cell line was the cloning of the I7L orf, by conventional, PCR-mediated methodology, into an E. coli expression plasmid and into an eukaryotic expression plasmid. Expression of the I7L-orf in E. coli allows rapid production of the protein for immunization of rabbits to obtain anti-I7L-protein antibodies, which can be later used to identify the I7L protein in permanent cell lines.

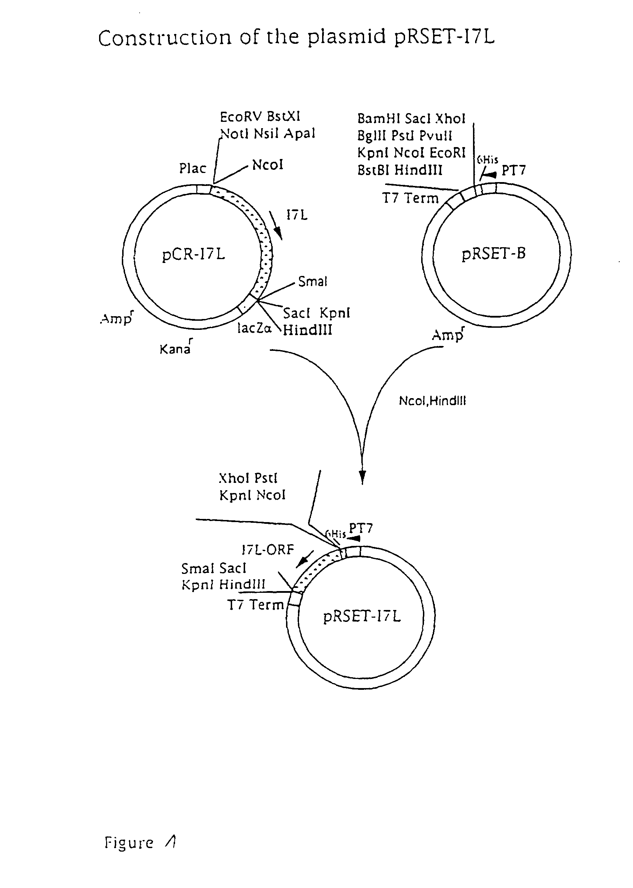

For this purpose, the I7L orf was amplified by PCR and then cloned into the plasmid pCRII (Invitrogen, Inc.). The primers oI7-1 (5'-AGT ACT CCA TGG AAA GAT ATA CAG ATT TAG-3') (SEQ ID NO:1) and oI7-2 (5'-ATC CCG GGT TTT AGA GAC TTT GAA GCT ACT-3') (SEQ ID NO:2) were used to amplify the I7L-gene as a 1.3kb fragment. This fragment was inserted into the plasmid pCRII, which resulted in pCR-I7L. Plasmid pCR-I7L was the source of the NcoI-HindIII gene fragment that was later inserted by forced cloning into the plasmid pRSET-B (InVitrogen, Inc.), yielding pRSET-I7L (Figure 1). Plasmid pRSET-I7L allowed over-expression of the I7L orf, which was downstream of the T7 promoter. Expression under the T7 promoter control requires T7 RNA polymerase, which is provided by infection of the plasmid-carrying E. coli by bacteriophage M13mp18/T7.

The I7L protein was over-expressed in JM109 E. coli cells infected with M13mp18/T7 and harboring plasmid pRSET-I7L. Induction of the lac promoter drives the expression of the T7 polymerase in the bacteriophage M13mp18/T7 with the isopropylthiogalactoside ("IPTG"). The E. coli expression system used in this particular example was obtained from InVitrogen, Inc. (USA), and includes the parental plasmids pRSET A, pRSET B and pRSET C and an M13-based helper phage M13mp18/T7. Other appropriate expression systems also can be used with the present invention.

Following expression, bacterial lysates were prepared and subjected to polyacrylamide gel electrophoresis. The band containing the I7L protein was detected and excised from the gel and electroeluted. Rabbits were immunized with the electroeluted material and complete Freund's adjuvant (30 mg protein per dose, i.m.). After one month, the animals are boosted with the same dose in incomplete Freund's adjuvant and development of antibodies is monitored by Western blots.

The Western blots may be performed essentially as described by Towbin et al., Proc. Nat'l Acad. Sci. USA 76: 4350-54 (1979). The first antibody is a rabbit anti I7L antibody (see above) used in a 1:100 dilution. The second antibody is a goat-anti-rabbit IgG coupled with alkaline phosphatase (BioRad, Inc.) used in a 1:1000 dilution. The reagents (BCIP and NBT) and staining protocols are from Promega, Inc.

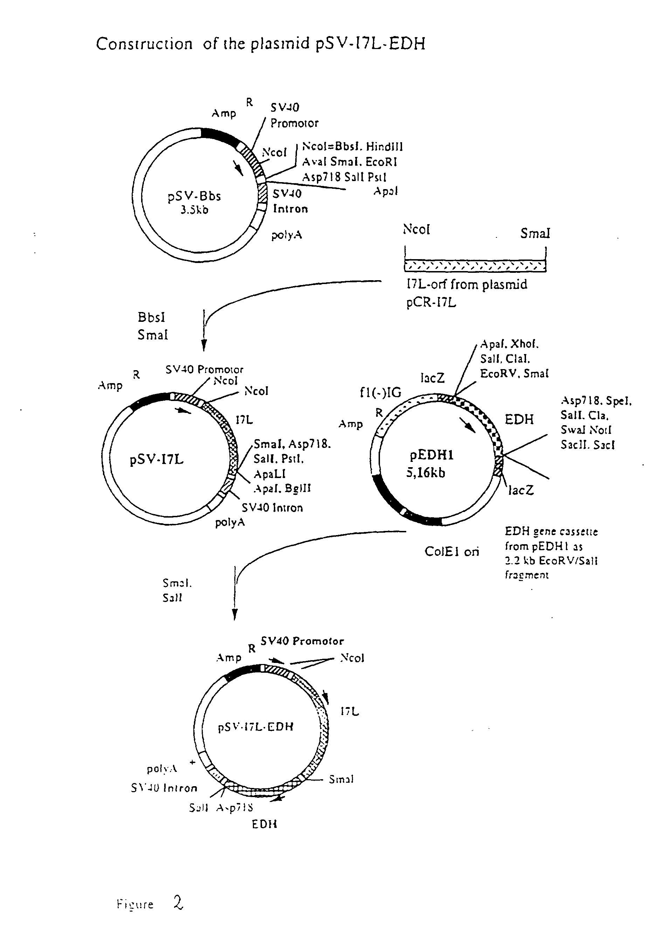

An NcoI-SmaI gene fragment from plasmid pCR-I7L is part of the expression plasmid pSV-I7L-EDH. See Figure 2. The plasmid pEDHl (Figure 2) comprises the 'EDH gene cassette' including an internal ribosome entry site, the dihydrofolate reductase ('dhfr') and hygromycin ('hph') genes as an EcoRV-SalI fragment. This plasmid pEDHl derives from the plasmid pB4/EDHPro (Herlitschka, thesis), in which four additional restriction sites (SalI, ClaI, SwaI and DraI) were inserted downstream of the 'EDH gene cassette' between the sites SpeI and NotI. The 'EDH gene cassette' of pEDH1 was inserted as an EcoRV-SalI fragment between the unique restriction sites SmaI and SalI of the plasmid pSV-I7L. This construction yielded plasmid pSV-I7L-EDH.

Plasmid pSV-I7L was obtained by inserting the 1.3kb NcoI-SmaI fragment of pCR-I7L between the BbsI-SmaI sites of plasmid pSV-Bbs. Plasmid pSV-Bbs is a derivative of the plasmid pSVβ (Clontech, Inc. USA), which lacks the β-galactosidase gene but contains a unique BbsI site. The BbsI site is introduced by a linker including the oligonucleotides oBbs-1 (5'-CTA GGC TTT TGC AAA AAG CTC CTC GAC CAT GGT GTC TTC A-3') (SEQ ID NO:3) and oBbs-2 (5'-AGC TTG AAG ACA CCA TGG TCG AGG AGC TTT TTG CAA AAG C-3') (SEQ ID NO:4). The linker was inserted between the AvrII and the HindIII sites of the plasmid pSVβ. See Figure 2.

In pSV-I7L-EDH, the I7L-orf is controlled by the SV40 early promoter. The selection marker for obtaining permanent cell lines is a fusion gene including the dhfr and hph genes, which allows efficient screening and amplification of genes of interest in permanent cell lines. Herlitschka, S. E. (1994), Ph.D. thesis, Universitat für Bodenkultur, Vienna, Austria. Practice of the present invention is not limited to the use of this marker, however.

This plasmid is transfected into monkey kidney Vero cells (ATCC No. CCL 81). Vero cells were obtained from the American Type Culture Collection (Rockville, MD). The cells were transfected with the plasmid pSV-I7L EDH according to Graham and van der Eb, Virol. 52: 456-67 (1973) and incubated in selective medium based on hygromycin B (DMEM, 10% fetal bovine serum, 250 mg/ml hygromycin B). Colonies became visible after 10 to 14 days. These colonies were expanded, subcloned twice and then characterized further. Permanent hph positive cell lines were selected in the presence of the hygromycin B. The cell lines were further characterized by PCR analysis for the presence of the complementing gene construct (I7L). Western blots are used to show that the gene of interest, the I7L-protein is expressed. The final complementing cell line is named V-I7L.

### Construction of Defective Virus d-I7L-ZG:

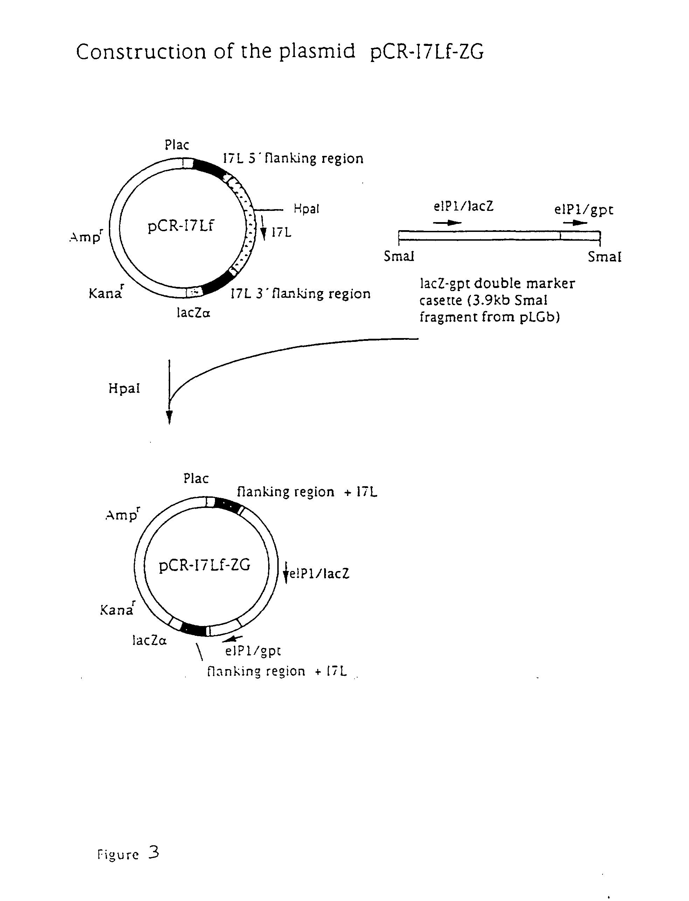

To construct the virus d-I7L-ZG, which lacks the I7L product, the plasmid pCR-I7Lf was constructed as summarized in Figure 3. This plasmid is based on the pCRII vector and contains the I7L-orf, including about 0.5 kb of flanking region on either side. The primers oI7-3 (5'-AGG AGT TAA TGA GGC CAA TGG A-3') (SEQ ID NO:5) and oI7-4 (5'-GAC ATA GGT ATA GAA TCC GGA-3') (SEQ ID NO:6) were used to obtain a PCR product of about 2.3 kb, which was subcloned into the pCRII plasmid to yield pCR-I7Lf.

A double gene cassette including the E. coli genes lac Z and gpt was excised as a SmaI fragment from the plasmid pLGb and inserted into the unique HpaI site located within the I7L-orf, which inactivates the pCR-I7L gene in the resulting plasmid pCR-I7Lf-ZG.

Plasmid pLGb was obtained from p2T, which is based on plasmid p1Ta and pTZ19R (Pharmacia). First, the large PvuII vector fragment of pTZ19R was ligated with annealed oligonucleotides P-1T(1): 5'- AGT TTA AAC GGC GCG CCC GGG CTC GAG AGG CCT CTG CAG ATG CAT CCA TGG GGA TCC GAA TTC 3' (SEQ ID NO:7) and P-1T(2): 5' - GAA TTC GGA TCC CCA TGG ATG CAT CTG CAG AGG CCT CTC GAG CCC GGG CGC GCC GTT TAA ACT (SEQ ID NO:8). This yielded p1Ta, which was then cleaved with EcoRI and BamHI. The cleaved plTa was then ligated to annealed oligonucleotides P2T(1): 5'-GAT CCT ACG TAT CTA GAA TTA ATT AAT GGC CAA TTT AAA TGC CCG GGA-3' (SEQ ID NO:9) and P-2T(2): 5'-AAT TTC CCG GGC ATT TAA ATT GGC CAT TAA TTA ATT CTA GAT ACG TAG-3' (SEQ ID NO:10). This yielded plasmid p2T, which was then cleaved with SmaI. A double gene cassette including the E. coli genes lacZ and gpt was inserted as a HindIII, Sal I and Klenow polymerase treated fragment. The double gene cassette is obtained from the plasmid pZgpt-a, which is based on plasmid pTNa and related plasmids.

For the construction of the d-I7L-ZG virus, plasmid pCR-I7Lf-ZG was inserted into the WR strain vaccinia virus by in vivo recombination in CV-1 cells. First, 5 x 106 CV-1 cells were infected with 0.1 pfu/cell of vaccinia WR, grown for 1 hour, transfected with a calcium phosphate precipitate that includes 20 µg of the plasmid pCR-I7Lf-ZG, and then grown for 3 days. See Graham and van der Eb, supra. A viral crude stock is prepared and used for plaque cells in the presence of gpt selection, Falkner and Moss, J. Virol. 62: 1849-54 (1988), and blue plaque screening, Chakrabarti et al., Mol. Cell. Biol. S. 5: 3403-09 (1985).

The defective virus grows in V-I7L cells only and is gpt and lacZ positive, while the wild-type virus grows in both cell lines, but is gpt and lacZ negative. The purification is performed ten times. The purified defective viruses are then grown to larger scale and examined by Southern blotting. The absence of wild-type virus and presence of the predicted bands confirms that the correct defective genomes have formed. Plaque assays of the defective viruses on the complementing cells and on wild-type cells confirm that the host range of the defective viruses are limited to the complementing cell line. Animal studies confirm that the defective viruses are non-pathogenic.

### Example 2: Construction of a defective vaccinia virus expressing the HIV env gp160 gene (d-I7L MN) in the complementing cell line V-I7L

To demonstrate foreign gene expression in the novel system, the HIV gpl60 gene of the HIV MN strain is inserted into the essential region I7L. For this purpose, a gene cassette from a SmaI fragment of plasmid pSep-ST2 is obtained. Plasmid pSep-ST2 contains the HIV-1 gpl60MN sequences controlled by the strong semi-synthetic poxvirus promoter Sep and a selection cassette that includes the P7.5 gpt gene. See Falkner and Moss, J. Virol. 62: 1849-54 (1988).

To construct the plasmid pSep-ST2, first the plasmid pSep(1) was constructed. To obtain pSep(1), the semi-synthetic poxvirus promoter Sep was constructed by combination of the late fowlpoxvirus promoter P2 (EP 0 538 496 Al, Dorner et al) with a synthetic early promoter sequence. Briefly, the HpaI/NcoI digested vector pS2gpt-P2 (European Patent Application Publication Number 0 561 034 A2) was ligated with the annealed oligonucleotides P-Sep(3) & P-Sep(4). The sequence of the oligonucleotides was, P-Sep(3), 5'-CTCGTAAAAA TTGAAAAACT ATTCTAATTT ATTGCACGGT CGCGA-3' (SEQ ID NO:11), and P-Sep(4), 5'-CATGGTACGT ACCGTGCAAT AAATTAGAAT AGTTTTTCAA TTTTTACGAG-3' (SEQ ID NO:12). The resulting plasmid was designated pSep(1). To obtain pSep-ST2, first a 2.65kb StuI/PvuII fragment derived from the plasmid pMNenv2 (described in EP 0 561 034 A2), containing the HIV1-MN env gene, was ligated with the SnaBI linearised plasmid pSep(1). The resulting plasmid, containing the insert in the proper orientation with respect to the "Sep-premoter" was designated pSep-gp160mn. In order to repair a point mutation located within the gp160-orf, a 1.9kb NsiI/SalI fragment of pSep-gp160mn was replaced by an equivalent fragment derived from the plasmid pMN-ST2. The resulting plasmid was designated pSep-ST2. The plasmids pMNenv1 and pMN-ST2 were provided by Marvin Reitz (N.C.I. Bethesda, Maryland, USA).

The cassette from pSep-ST2 contains the HIV gpl60 gene and E. coli gpt gene, and is inserted into the unique HpaI site of pCR-I7Lf resulting in the plasmid pCR-I7Lf-MN. This plasmid is used in an in vivo recombination experiment in CV-1 cells involving WR wild type virus to obtain a viral crude stock that is a mixture of: (1) defective viruses, which have undergone two cross-over events to become a recombinant virus; (2) viruses that have undergone only one cross-over event and (3) wild-type helper viruses. An overall explanation of cross-over events is contained in Falkner and Moss, J. Virol. 64: 3108-11 (1990).

This mixture of viruses is plaque purified in the complementing cell checked for purity as described in Example 1, except that the plaque assays are based solely on gpt-selection. The resulting virus is designated d-I7L-MN and used for expression of recombinant gpl60 in V-I7L cells. The virus d-I7L-MN is used in combination with its complementing cell line to express gpl60. V-I7L-cells are infected with 0.1 pfu of d-I7L-MN and grown for three days. The recombinant protein is detected by Western blots using an anti-gp41 monoclonal antibody in a method according to Towbin et al., supra. Animal experiments in mice are carried out to demonstrate that d-I7L-MN is non-pathogenic but still able to prime immune response.

### Example 3: Construction of a defective vaccinia virus lacking the large subunit of the early transcription factor (d-A8L-ZG) and a complementing cell line for propagating the virus

### Construction of a helper cell line based on Vero cells (V-A8L) :

The VETF factor is a heterodimer comprised of a 77kD polypeptide and a 82kD polypeptide, which are encoded respectively by the D6R and the A8L orfs of the vaccinia-WR genome. Gershon and Moss, Proc. Nat'l Acad. Sci. USA 87: 4401-05 (1990). VETF proteins are expressed late in infection and packaged into the nascent virus particles. A defective virus lacking VETF is desirable because it should be able to perform an additional life cycle in wild-type hosts as compared to viruses capable only of performing abortive life cycles.

The D6R subunit contains a possible ATP binding site and has been found to bind to the early promotor DNA sequence. See Broyles and Li, J. Virol 67: 5677-80 (1993). Temperature sensitive mutations have been mapped to the D6R gene. The A8L orf was chosen in order to avoid undesirable protein-DNA interactions in the complementing cell line.

The first step in the construction of the helper cell line was cloning of the A8L orf by PCR mediated techniques into an E. coli expression plasmid and into an eukaryotic expression plasmid. Expression of the orf in E. coli allowed rapid production of the protein for immunization of rabbits to obtain anti-VETF large subunit antibodies that are subsequently used to identify the protein in the permanent cell lines.

For this purpose, the A8L-orf was amplified by PCR and the cloned into the plasmid pCRII. The primers oA8-9t (5'-AAA CTG CAG GAT GCG ATA TAT AGT AAG TCC GCA AAT GGT ATT A-3') (SEQ ID NO:13) and oA8-2t (5'-AGG AAT TCA GGC CTG TTT AAT TAA TTT GTG CTC TTC-3') (SEQ ID NO:14) were used to amplify the A8L-gene as a 2.1 kb fragment. This fragment was inserted into the plasmid pCRII (InVitrogen Inc.), resulting in pCR-A8Lt.

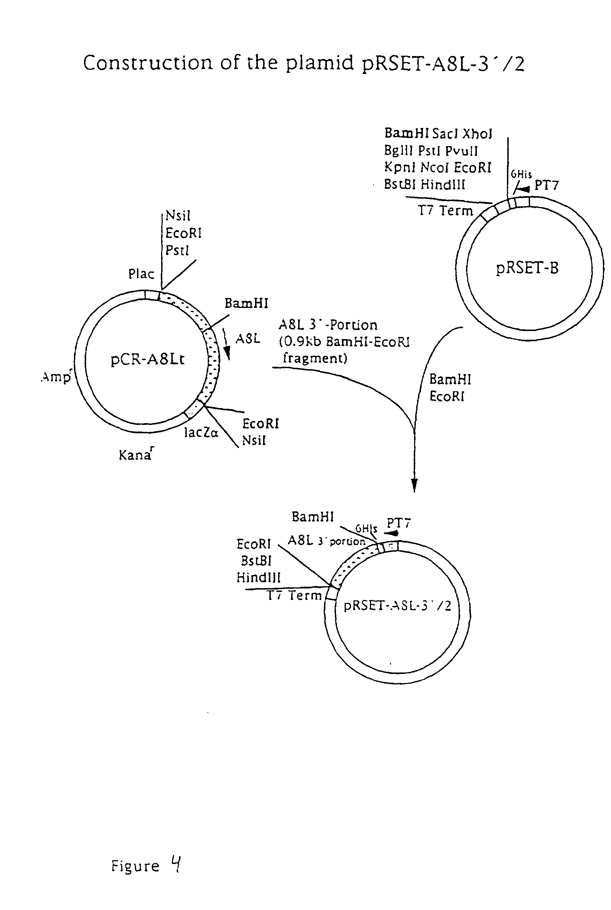

Plasmid pCR-A8Lt is the source of a 0.9 kb BamHI-EcoRI gene fragment. The BamHI site (present in the A8L gene) and the EcoRI site (introduced by the multiple cloning site of pCRII) were cleaved to obtain the fragment which was inserted by forced cloning into the plasmid pRSET-B (InVitrogen, Inc.) resulting in the plasmid pRSET-A8L-3'/2. See Figure 4. The plasmid contains a fusion gene encoding the C'-terminal portion of the VETF large subunit with a N'-terminal 6-histidine-tag. The truncated A8L-protein was over expressed in E. coli cells (strain JM109) harboring the plasmid pRSET-A8L-3'/2.

The bacteria were thereafter infected by the helper phage M13mp18/T7 in presence of IPTG, which induces the lac promoter driving the expression of T7 polymerase in the bacteriophage M13mp18/T7. The recombinant truncated A8L-protein including the his-tag was purified from bacterial lysates by affinity chromatography on nickel-NTA columns, according to the manufacturer's protocol (Diagen, Inc.). The purified material emulsified in complete Freund's adjuvant was used to immunize rabbits (80 mg protein per dose, s.c. and i.m.). After one month, the animals were boosted with the same dose and development of antibodies was monitored by Western blots.

To characterize the rabbit anitsera the A8L-proteins in bacterial lysates were detected by Western blots. The Western blots were done essentially as described by Towbin et al., supra. The first antibody was a rabbit anti-A8L protein antibody used in a 1:100 dilution. The second antibody was a goat-anti-rabbit IgG coupled with alkaline phosphatase (BioRad, Inc.) used in a 1:1000 dilution. The reagents (BCIP and NBT) and staining protocols were from Promega, Inc.

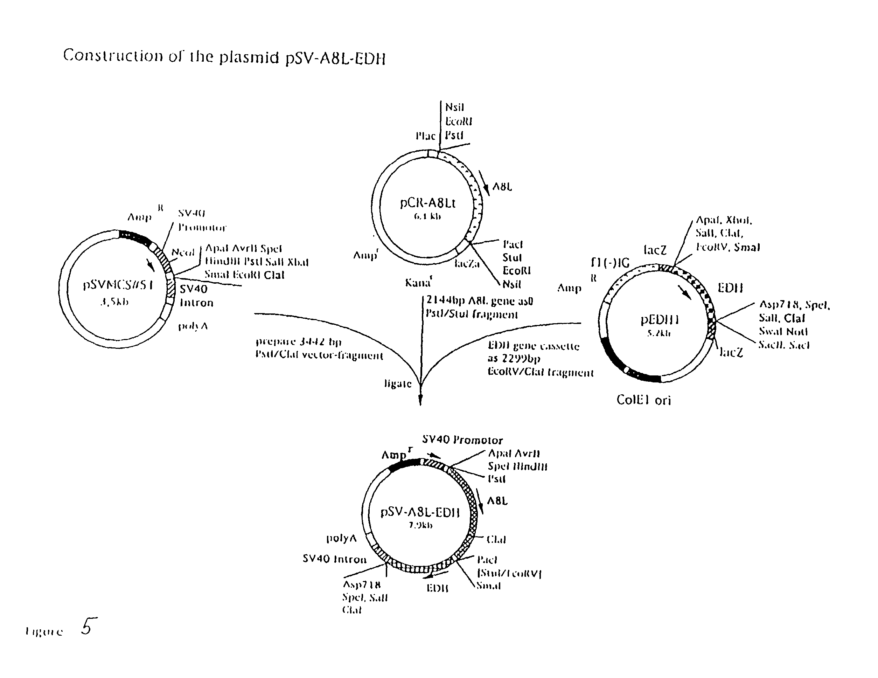

To obtain the expression plasmid pSV-A8L-EDH the A8L gene was isolated from the plasmid pCR-A8Lt as a 2.1kb PstI-StuI gene fragment. The sites PstI and StuI were introduced by the PCR primers oA8-9t and oA8-2t, respectively. This fragment and a EcoRV-ClaI gene fragment from the plasmid pEDH1 comprising the "EDH gene cassette" were ligated into the PstI-ClaI cut expression vector pSVMCS#51, which is derived from pSVβ (Clontech, Inc.) by substitution of the β-galactosidase gene by a multiple cloning site ("msc") including the restriction sites ApaI, AvrIII, SpeI, HindIII, PstI, SalI, XbaI, SmaI, EcoRI and ClaI. For this purpose the vector pCMVβ was cut with NotI and religated resulting in the plasmid pCMV. This plasmid was cut with SalI and HindIII, treated with Klenow polymerase and religated resulting in an intermediate plasmid. This plasmid was cut with XhoI and a double stranded linker, coding for the restriction sites ApaI, AvrIII, SpeI, HindIII, PstI, SalI, XbaI, SmaI, EcoRI and ClaI was inserted resulting in the plasmid pSVMCS#51. See Figure 5.

In plasmid pSV-A8L-EDH, the A8L-orf is controlled by the SV40 early promoter. The selection marker for obtaining permanent cell lines is a fusion gene including the dhfr gene and the hph gene as described in Example 1.

The plasmid pSV-A8L-EDH was transfected into monkey kidney Vero cells (ATCC No. CCL 81) in a method according to Graham & van der Eb, supra, and was incubated in selective medium (DMEM, 10% fetal bovine serum, 250 µg/ml hygromycin) and further treated as described in Example 1. Permanent hygromycin phosphotransferase positive cell lines were selected in the presence of the antibiotic hygromycin B. After five passages integration of the foreign DNA was demonstrated by PCR using primers specific for a 0.5kb segment of the A8L gene.

Western blots are used to determine whether the gene of interest, the VETF large subunit, is expressed. The cell lines expressing the highest levels of VETF large subunit is further characterized by PCR analysis and used as the complementing cell line and named V-A8L.

### Construction of the defective virus d-A8L-ZG:

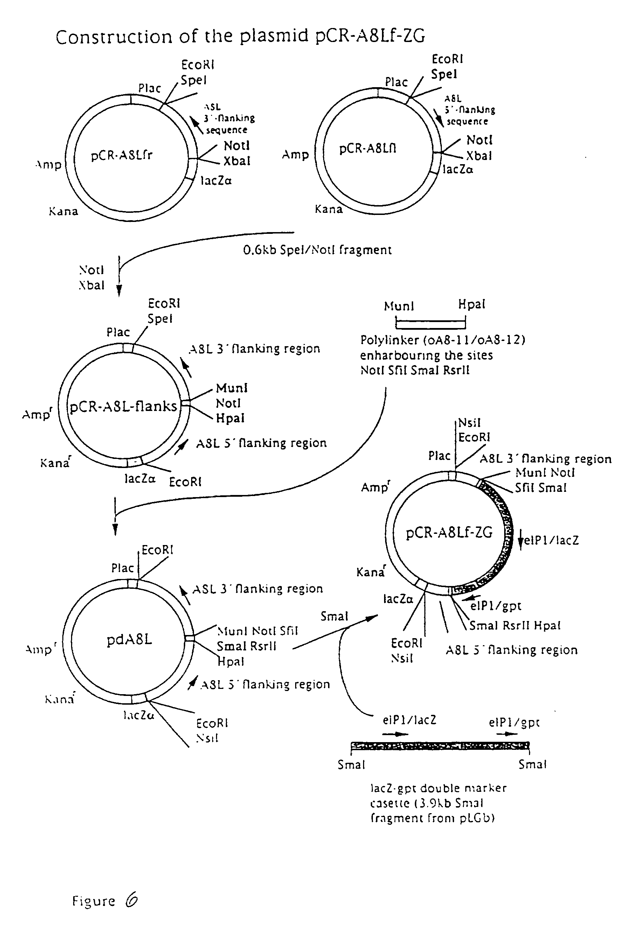

To construct the virus d-A8L-ZG the plasmid pCR-A8Lf-ZG was cloned as schematically shown in Figure 6. Regions (sized between 0. 5kb and 0. 7kb) flanking the A8L orf at either sides were amplified by PCR and subcloned into the plasmid pCRII to obtain pCR-A8L-fl and pCR-A8L-fr respectively.

The PCR-primers for the amplification of the A8L 5'-flanking region were oA8-8 (5'-AGC GCC GCT ACT AGC ACT CC-3') (SEQ ID NO:15) and oA8-5 (5'-GAA CGT TAA CAT TTA TAT CGT GGG GTA AAG TGA AAA TC-3')(SEQ ID NO:16). The primers for the A8L 3'-flanking region were oA8-4 (5'- TTG GAG AAC TTG ATA CGC CG-3') (SEQ ID NO:17) and oA8-6 (5'-CAT ATG CAA TTG TAA ATT AAA CAA CTA AAT CTG TAA ATA AAT A-3') (SEQ ID NO:18). The A8L 5'-flanking region as 0. 7kb NotI-SpeI fragment from pCR-A8L-fl was ligated between the sites NotI and XbaI right upstream of the A8L 3'-flanking region in pCR-A8L-fr yielding the plasmid pCR-A8L-flanks.

In order to facilitate further cloning procedures, a synthetic linker containing the rare restriction sites SmaI, NotI, SfiI and RsrII was ligated between the A8L-flanking regions in pCR-A8L-flanks. The oligonucleotides used for the construction of the linker were oA8-11 (5'-AAC GGT CCG GCC CGG GCG GCC GCC-3') (SEQ ID NO:19) and oA8-12 (5'-AAT TGG CGG CCG CCC GGG CCG GAC CGT T-3')(SEQ ID NO:20). The plasmid pCR-A8L-flanks was thereafter linearized with MunI and HpaI (both sites are introduced by the PCR primers oA8-5 and oA8-6, respectively). The resulting plasmid, designated pdA8L, was ligated with a 3.9kb SmaI double gene cassette from the plasmid pLGb described in Example 1 to yield pCR-A8Lf-ZG. In this construct, the A8L orf is deleted completely in order to prevent rescue of the defective virus by the complementing cell line via homologous recombination.

For the construction of the d-A8L-ZG virus the plasmid pCR-A8L-ZG was inserted into the vaccinia virus WR. The plasmid pCR-A8Lf-ZG was used for in vivo recombination in CV-1 cells. First, 5 x 106 CV-1 cells were infected with 0. 1 pfu/cell of vaccinia WR, incubated for 1 hour, transfected with a calcium phosphate precipitate, according to Graham & van der Eb, supra, which contained 20 µg of the plasmid pCR-A8L-ZG and grown for 3 days.

This resulted in a crude stock containing wild-type viruses (helper) and defective viruses. To obtain pure stocks of the defective virus, plaque assays were carried out in V-A8L-cells that are able to complement the defective virus. The viral crude stock was prepared and used for plaque assays on V-A8L cells in the presence of gpt selection (Falkner and Moss (1988), supra) and blue plaque screening (Chakrabarti et al., supra). The plaque purification is repeated ten times. The purified defective poxviruses are grown to larger scale and examined by Southern blotting. The absence of wild-type virus and presence of the predicted bands confirms that the correct defective genomes have formed. Plaque assays of the defective viruses on the complementing cells and on wild-type cells confirm that the host range of the defective viruses are limited to the complementing cell line. Animal studies confirm that the defective viruses are non-pathogenic.

### Example 4: Construction of a defective vaccinia virus lacking the small subunit of the early transcription factor (d-D6R-ZG) and of a complementing cell line

### Construction of a helper cell line based on Vero cells (V-D6R):

The small subunit of the early transcription factor VETF is a polypeptide of 77kD in size. As described in Example 3, VETF contains a possible ATP binding site and has been found to bind to the early promotor DNA sequence. Broyles and Li, supra. The D6R orf is chosen for complementation because temperature sensitive mutations have been mapped to this gene, which is an element of the definition of an essential gene. Seto et al., Virol. 160: 110-19 (1987).

The steps in the construction of the helper cell line V-D6R are in general identical to the construction of the cell line V-A8L in Example 3. First the D6R orf was cloned into the plasmid pCRII (InVitrogen, Inc.) by PCR mediated techniques. The resulting plasmid is the source of the D6R gene fragment for the construction of an eukaryotic expression vector and an E. coli expression plasmid. Expression of the orf in E. coli allows rapid production of the protein for immunization of rabbits to obtain anti-VETF small subunit antibodies that are subsequently used to identify the protein in the permanent cell lines.

For this purpose, the D6R-orf was amplified as a 1.9 kb fragment by PCR using the primers oD6-1 (5'-CTG CAG AAT GAA TAC CGG AAT TAT AGA TTT-3') (SEQ ID NO:21) and oD6-2 (5'-CCC GGG TTA TGG AGA AGA TAC CAC GTT-3') (SEQ ID NO:22), and cloned into pCRII resulting in the plasmid pCR-D6R.

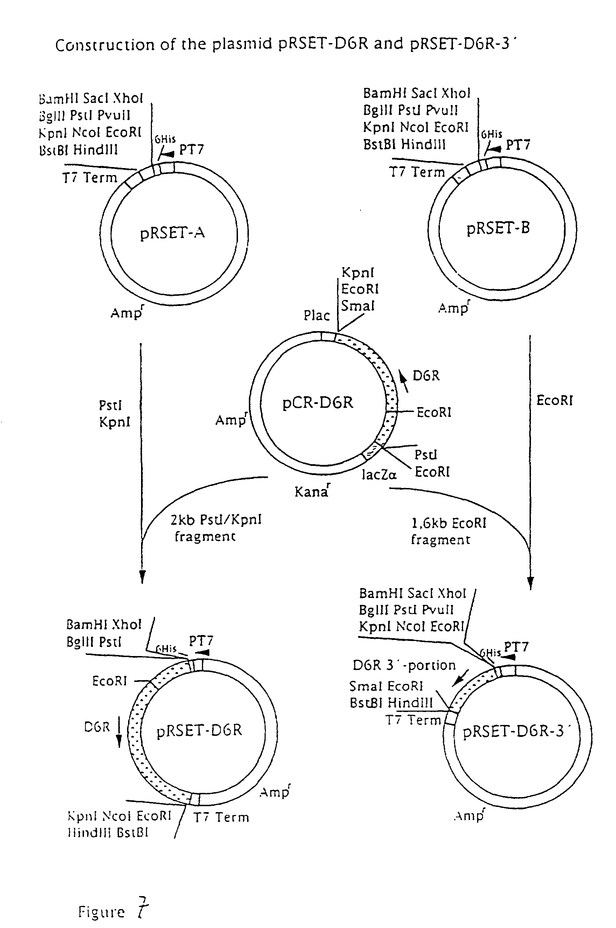

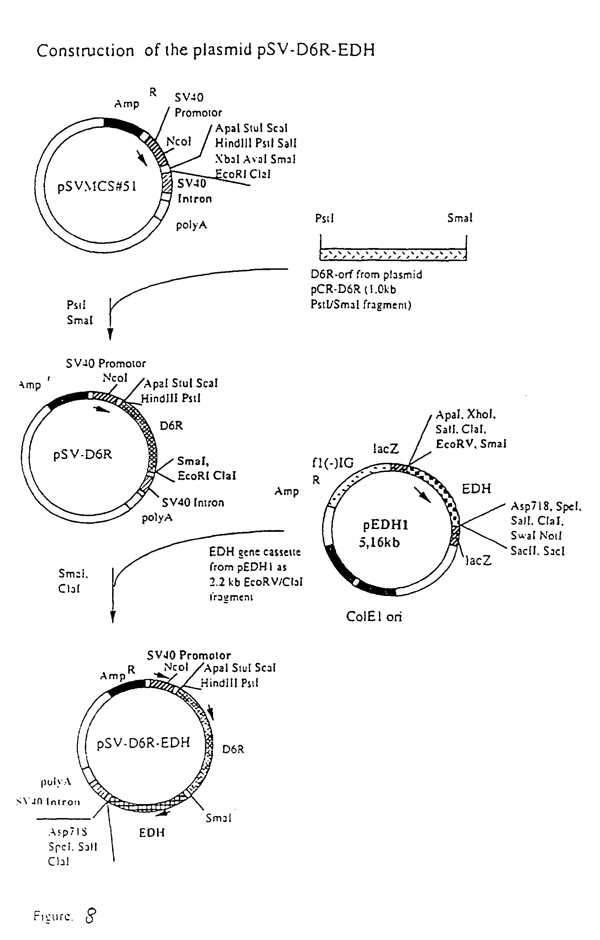

As shown in Figure 7, the E. coli expression plasmid pRSET-D6R-3' was constructed by ligating a 1.6 kb EcoRI fragment from pCR-D6R into the vector pRSET-B (InVitrogen, Inc.). Plasmid pRSET-D6R-3' contains a fusion gene encoding a N'-terminal 6-histidine-tag fused to the VETF small subunit lacking its first 100 amino acids. Bacterial expression, the tag-mediated purification of the recombinant protein and the production of anti-VETF small subunit antibodies were performed as outlined in Example 3. The expression plasmid pSV-D6R-EDH was constructed as follows: The 1.9kb gene fragment D6R orf was isolated from pCR-D6R by cutting the plasmid with PstI and SmaI and ligated into the vector pSVMCS#51 (Herlitschka, supra) by forced cloning to obtain the plasmid pSV-D6R. The sites PstI and SmaI were introduced by the PCR primers oD6-1 (5'-CTGCAGAATG AATACCGGAA TTATAGATTT -3') (SEQ ID NO:21) and oD6-2 (5'-CCCGGGTTAT GGAGAAGATA CCACGTT-3') (SEQ ID NO:22), respectively. Ligation of an EcoRV-ClaI gene fragment from the plasmid pEDHl comprising the "EDH gene cassette" into SmaI-ClaI cut pSV-D6R yielded the expression plasmid pSV-D6R-EDH. See Figure 8.

In plasmid pSV-D6R-EDH, the D6R-orf is controlled by the SV40 early promoter. The selection marker for obtaining permanent cell lines is a fusion gene having the dhfr gene and the hph gene as described in Example 1. Transfection and screening procedures for the construction of the complementing cell line are identical to those in Example 3.

### Construction of the defective virus d-D6R-ZG:

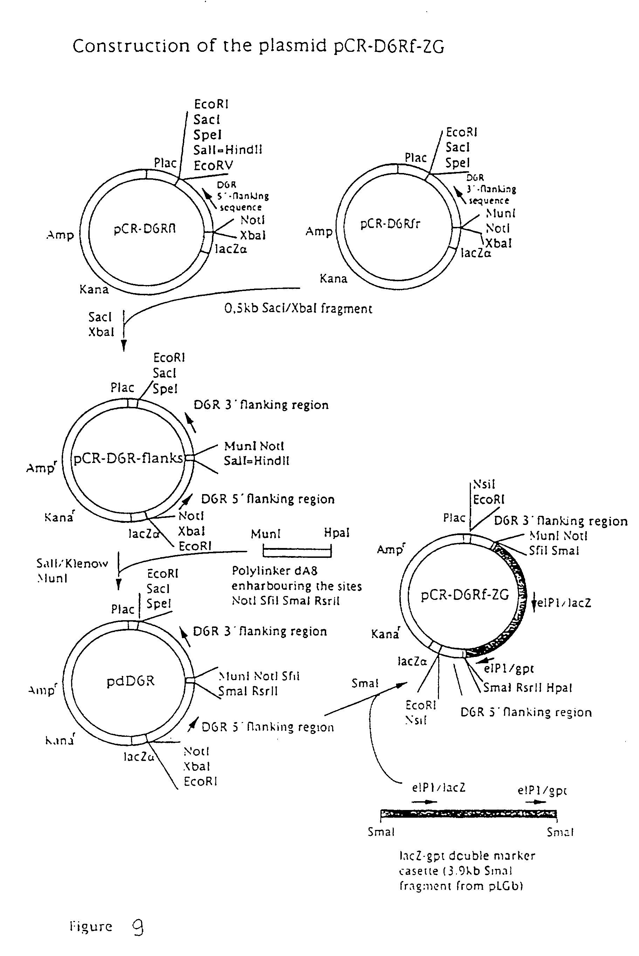

To construct the virus d-D6R-ZG the plasmid pCR-D6Rf-ZG was cloned as schematically shown in Figure 9. Regions (sized between 0.5 kb and 0.6 kb) flanking the D6R orf at either sides were amplified by PCR mediated techniques and subcloned into the plasmid pCRII to obtain pCR-D6R-fl and pCR-D6R-fr, respectively. The PCR-primers for the amplification of the D6R 5'-flanking region are oD6-3 (5'-TGA GAA GAA TTG CCG TCG-3') (SEQ ID NO:23) and oD6-5 (5'-GTC GAC GAT ATC TTC TAT AAA TAT ATG AGC-3') (SEQ ID NO:24). The primers for the D6R 3'-flanking region are oD6-4 (5'-TAG AGT ACC CGA ATC TCT AC-3') (SEQ ID NO:25) and oD6-6 (5'-ACA ATT GGT ATT PAG TAT AAC GAC TCC-3') (SEQ ID NO:26). The D6R 5'-flanking region as 0.6kb SacI-SpeI fragment from pCR-D6R-fl was ligated between the sites SacI and XbaI right downstream to the D6R 5'-flanking region in pCR-D6R-fl yielding the plasmid pCR-D6R-flanks. In order to facilitate further cloning procedures a synthetic linker containing the rare restriction sites SmaI, NotI, SfiI and RsrII was ligated between the D6R-flanking regions in pCR-D6R-flanks. The oligonucleotides used for the construction of the linker are oA8-11 and oA8-12. See Example 3. The plasmid pCR-D6R-flanks is therefore linearized with SalI, treated with Klenow Polymerase and cut with MunI (both restriction sites are introduced by the PCR primers oD6-5 and oD6-6, respectively). The resulting plasmid designated pdD6R is ligated with a 3.9kb SmaI double gene cassette from the plasmid pLGb described in Example 1 to yield pCR-D6Rf-ZG. In this construct, the D6R orf is deleted completely in order to prevent rescue of the defect virus by the complementing cell line due to homologous recombination.

For the construction of the d-D6R-ZG virus the plasmid pCR-D6RF-ZG is inserted into the vaccinia virus WR. The plasmid pCR-D6Rf-ZG is used for in vivo recombination in CV-1 cells. First, 5 x 106 CV-1 cells are infected with 0.1 pfu/cell of vaccinia WR, incubated for 1 hour, transfected with a calcium phosphate precipitate (according to Graham & van der Eb, supra) containing 20 µg of the plasmid pCR-D6R-ZG and grown for 3 days. This results in a crude stock containing wild-type viruses (helper) and defective viruses. To obtain pure stocks of the defective virus, plaque assays are carried out in V-D6R-cells that are able to complement the defective virus. The viral crude stock is prepared and used for plaque assays on V-D6R cells in the presence of gpt selection (Falkner and Moss, supra (1988) and blue plaque screening (Chakrabarti et al., supra). The plaque purification is repeated ten times. The purified defective poxviruses are grown to larger scale and examined by Southern blotting. The absence of wild-type virus and presence of the predicted bands confirms that the correct defective genomes have formed. Plaque assays of the defective viruses on the complementing cells and on wild-type cells confirm that the host range of the defective viruses are limited to the complementing cell line. Animal studies confirm that the defective viruses are non-pathogenic.

### Example 5: Construction of a defective vaccinia virus lacking the J1R gene product (d-J1R-ZG) and of a complementing cell line

### Construction of a helper cell line based on Vero cells (V-J1R):

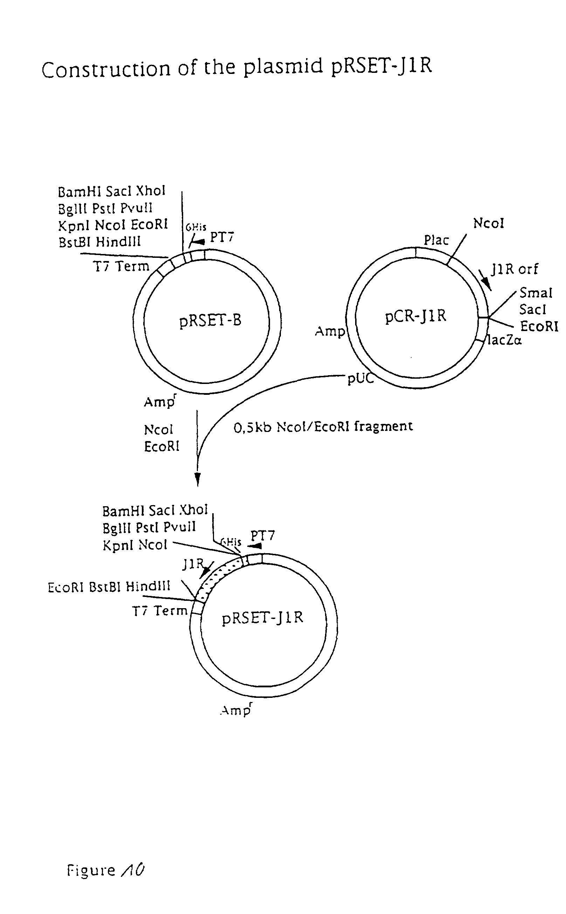

The first step was expression of the J1R orf in E. coli (using the InVitrogen pRSET-vector system). First, the plasmid pCR-J1R was constructed by cloning the J1R PCR product obtained with the primers oJ1R-1 (5'-GCCATGGATC ACAACCAGTA TCTCTT-3') (SEQ ID NO:27) and oJ1R-2 (5'-ACCCGGGTTA ATTPATTATT GTTCACTTTA-3') (SEQ ID NO:28) into the pCRII vector. An NcoI-EcoRI fragment was then inserted into pRSET-B yielding the E. coli expression vector pRSET-J1R (Figure 10). With this plasmid, high amounts of J1R protein were obtained which, notably, were not immunogenic in rabbits. Antibodies for the further identification of the protein in the permanent cell lines were thus raised against a conjugate of a synthetic J1R peptide and the carrier protein KLH. The synthetic peptide with the amino acid sequence SLATTAIDPIRYIDP, corresponding to amino acid positions 118 to 132 of the J1R protein, was coupled to KHL (preactivated KLH from Pierce, USA). The purified conjugate in complete Freund's adjuvant was used to immunize rabbits (100 mg of protein per dose, s.c. and i.m.). After one month, the animals were boosted with the same dose and development of antibodies are monitored by Western blots.

Proteins are detected by Western blots. The Western blots are done essentially as described by Towbin et al., supra. The first antibody is a rabbit anti-J1R protein antibody used in a 1:100 dilution. The second antibody is a goat-anti-rabbit IgG coupled with alkaline phosphatase (BioRad, Inc.) used in a 1:1000 dilution. The reagents (BCIP and NBT) and staining protocols are from Promega, Inc (USA).

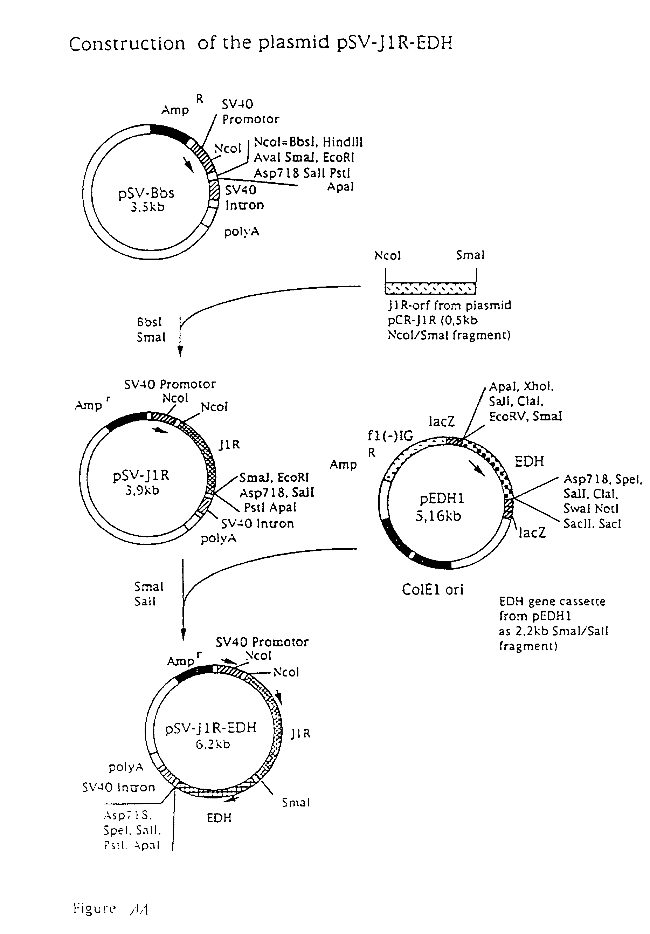

To obtain the expression plasmid pSV-J1R-EDH (Fig. 11) for transformation into the Vero cells a 0.5kb NcoI- SmaI gene fragment encoding the complete J1R gene was isolated from the plasmid pCR-J1R. The sites NcoI and SmaI were introduced by the PCR primers oJ1R-1 and oJ1R-2 (see above), respectively. This fragment was ligated into the BbsI cut expression vector pSV-Bbs (see Example 1). Insertion of a EcoRV-ClaI gene fragment from plasmid pEDH1 comprising the "EDH gene cassette" into SmaI-ClaI cut pSV-J1R yielded pSV-J1R-EDH. See Figure 11. In plasmid pSV-J1R-EDH the J1R-orf is controlled by the SV40 early promoter. The selection marker for obtaining permanent cell lines is a fusion gene including the dhfr gene and the hph gene as described in Example 1.

The plasmid pSV-J1R-EDH was transfected into monkey kidney Vero cells (ATCC No. CCL 81) in a method according to Graham & van der Eb, supra, and incubated in selective medium (DMEM, 10% fetal bovine serum, 250 µg/ml hygromycin) and further treated as described in Example 1. Permanent hygromycin phosphotransferase positive cell lines were selected in the presence of the antibiotic hygromycin B. After five passages integration of the foreign DNA was demonstrated by PCR using primers specific for a 0.5kb segment of the J1R gene. Western blots are used to determine whether the gene of interest, the J1R protein, are expressed. The cell lines expressing the highest levels of J1R protein is further characterized by Southern blotting and used as the complementing cell line and named V-J1R.

### Construction of the defective virus d-J1R-ZG:

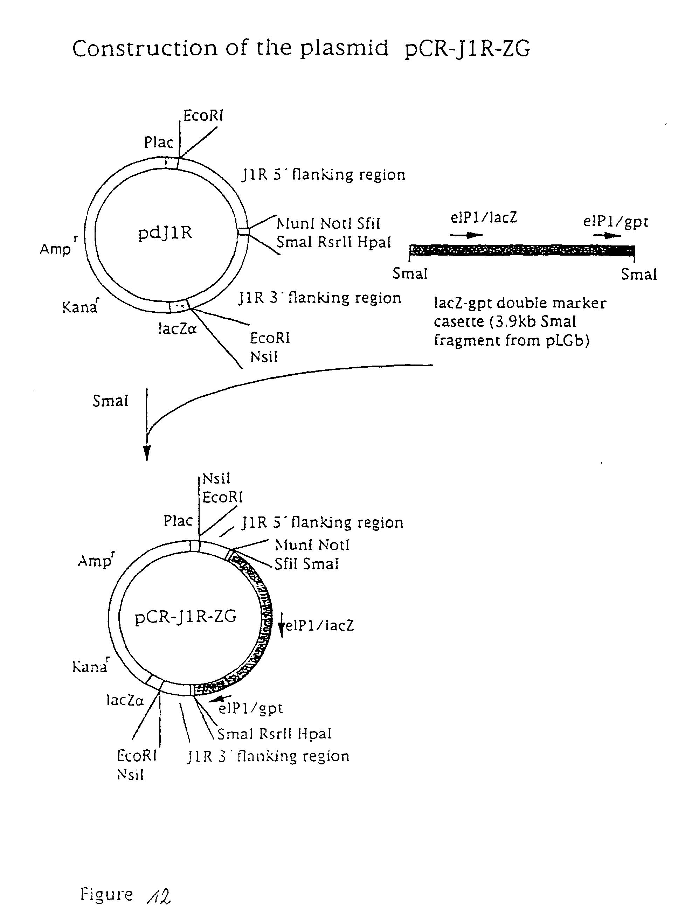

To construct the virus d-J1R-ZG the plasmid pCR-J1Rf-ZG was cloned as schematically shown in Figure 12. A 1.5 kb gene fragment comprising the J1R orf and about 0.5kb of flanking sequences at either sides was amplified from vaccinia virus (strain WR) by PCR and subcloned into the plasmid pCRII to obtain pCR-J1Rf.

The PCR-primers were oJ1-3 (5'-TCC ACA TCC ATG AGA TTG AAT-3') (SEQ ID NO:29) and oJ1-4 (5'-CGA TTG ATA CAT ATC ATT ACC-3') (SEQ ID NO:30). Deletion of the complete J1R gene and the first 40 nucleotides of the adjacent thymidine kinase gene was also performed by PCR mediated techniques. The complete plasmid pCR-J1Rf, excluding the sequences to be deleted, was amplified via PCR resulting in a 6kb product. Polynucleotide kinase treatment and subsequent re-ligation of the PCR product yielded the plasmid pCR-J1R-flanks. The primers oJ1-5 (5'-AACAATTGCA TCATCTGCGA AGAACATCG-3') (SEQ ID NO:31) and oJ1-6 (5'-CAGTTAACTT TTCAGGTAAA AGTACAGAAT-3') (SEQ ID NO:32) that were employed for this reaction introduce the restriction sites MunI and HpaI. The plasmid was cleaved at these sites and the linker (oA8-11 and OA8-12 as described in Example 3) was inserted to obtain pdJIR. Analogous to Example 3 a lacZ-gpt double marker cassette was ligated into the SmaI site of pdJ1R resulting in the recombination vector pCR-J1Rf-ZG. (This plasmid only contains 40 base pairs of the J1R gene, at its 5' terminus, which are necessary for the integrity of the adjacent L5R orf in the defective virus.)

For the construction of the d-J1R-ZG virus the plasmid pCR-J1R-ZG was inserted into the vaccinia virus WR. The plasmid pCR-J1Rf-ZG was used for in vivo recombination in CV-1 cells. First, 5 x 106 CV-1 cells were infected with 0.1 pfu/cell of vaccinia WR; incubated for 1 hour, transfected with a calcium phosphate precipitate (according to Graham & van der Eb, supra) containing 20 micrograms of the plasmid pCR-J1R-ZG and grown for 3 days.

This procedure resulted in a crude stock containing wild-type (helper viruses) and defective viruses. To obtain pure stocks of the defective virus, plaque assays were carried out in V-JIR-cells that are able to complement the defective virus. The viral crude stock was prepared and used for plaque assays on V-J1R cells in the presence of gpt selection (Falkner and Moss (1988), supra) and blue plaque screening (Chakrabarti et al., supra), alternating with plaque assays on tk-negative V-J1R cells in the presence of BUdR (Mackett et al, supra). The plaque purification is repeated ten times. The purified defective poxviruses are grown to larger scale and examined by Southern blotting. The absence of wild-type virus and presence of the predicted bands confirms that the correct defective genomes have formed. Plaque assays of the defective viruses on the complementing cells and on wild-type cells confirm that the host range of the defective viruses are limited to the complementing cell line. Animal studies confirm that the defective viruses are non-pathogenic.

### Example 6: Construction of a defective vaccinia virus lacking the H4L gene product (d-H4L-ZG) and of a complementing cell line

The H4L orf is chosen for complementation as conditional lethal temperature sensitive mutations have been mapped to this gene. Seto et al., supra.

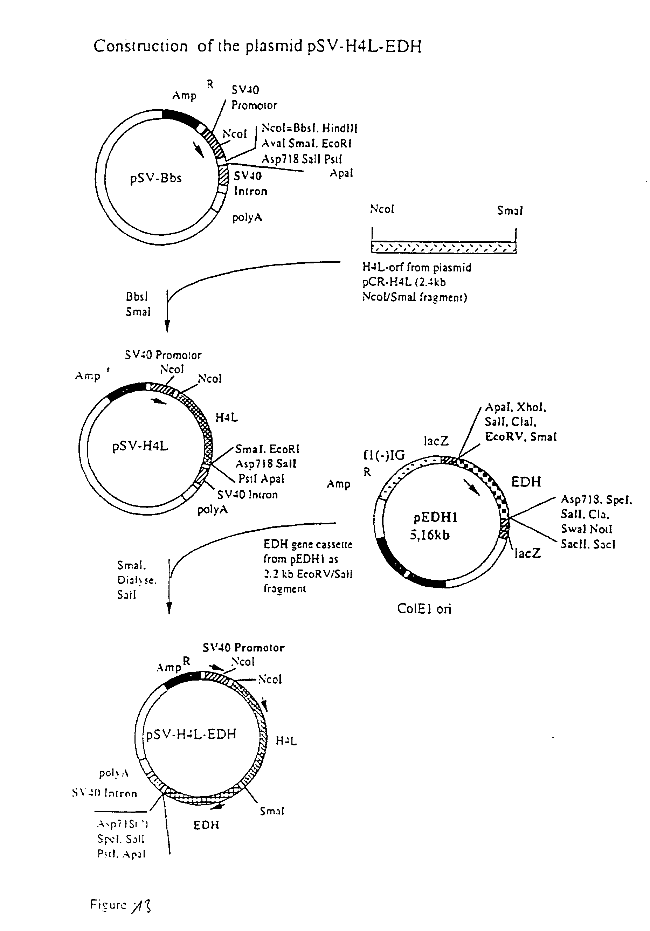

The steps in the construction of the helper cell line V-H4L are in general identical to the construction of the cell line V-A8L in Example 3. First, the H4L orf was cloned into the plasmid pCRII (InVitrogen, Inc.) by PCR mediated techniques. The resulting plasmid is the source of the H4L gene fragment for the construction of the Vero cell expression vector pSV-H4L-EDH as shown in Figure 13. Antibodies to identify the H4L gene product (RAP94) in the permanent cell lines are raised against a KLH conjugate of a synthetic peptide. The conjugate was prepared by linkage of the synthetic peptide KIYKNSFSEDHNNSLD corresponding to amino acid positions 242 to 258 in the H4L orf, Kane and Shuman, J. Virol. 66: 5752-62 (1992), to activated KLH (KLH coupling Kit, Pierce Inc., USA). This conjugate was used for the production of anti-H4L antibodies analogous to Example 5.

For the construction of the Vero cell expression vector, the H4L-orf was amplified as a 2.4kb fragment by PCR using the primers oH4-1 (5'-ACC ATG GAC TCT AAA GAG ACT AT-3') (SEQ ID NO:33) and oH4-2 (5'-CAA TTG CCC GGG TTA ATT AAT GAA ATT AAT CAT ATA CAA CTC-3') (SEQ ID NO:34) and cloned into pCRII resulting in the plasmid pCR-H4L. The expression plasmid pSV-H4L-EDH was constructed as follows: The H4L orf was isolated as a 2.4kb gene fragment from pCR-H4L by cutting the plasmid with NcoI and SmaI and ligated into the vector pSV-Bbs (see Example 1) by forced cloning to obtain the plasmid pSV-H4L. The sites NcoI and SmaI were introduced by the PCR primers oH4-1 and oH4-2, respectively. Ligation of an EcoRV-SalI gene fragment from the plasmid pEDH1 comprising the "EDH gene cassette" into Smal-SalI cut pSV-H4L yielded the expression plasmid pSV-H4L-EDH. See Figure 13.

In plasmid pSV-H4L-EDH, the H4L-orf is controlled by the SV40 early promoter. The selection marker for obtaining permanent cell lines is a fusion gene including the dhfr gene and the hph gene as described in Example 1. Transfection and screening procedures for the construction of the complementing cell line were done as described in Example 3, except that H4L-specific reagents were used.

### Construction of the defective virus d-H4L-ZG:

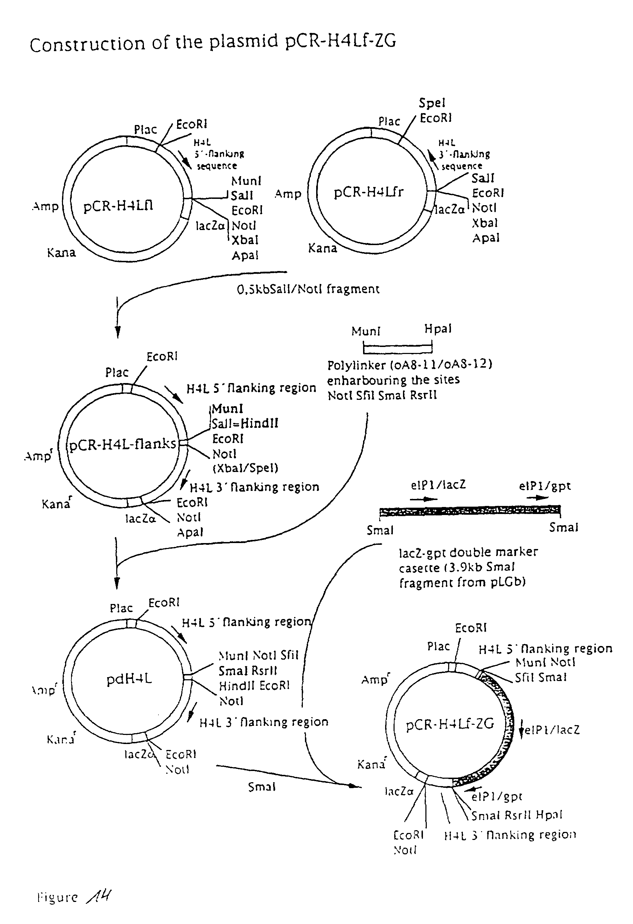

To construct the virus d-H4L-ZG, the plasmid pCR-H4Lf-ZG was cloned as schematically shown in Figure 14. Regions (sized between 0.5kb and 0.6kb) flanking the H4L orf at either sides were amplified by PCR mediated techniques and subcloned into the plasmid pCRII to obtain pCR-H4L-fl and pCR-H4L-fr, respectively.

The PCR-primers for the amplification of the H4L 5'-flanking region are oH4-3 (5'-CCT TTA GGT CAG AA GAT CGC-3') (SEQ ID NO:35) and oH4-5 (5'-GTC GAC AAT TGT TAA AG TAC AAA CAA CTA GGA-3') (SEQ ID NO:36). The primers for the H4L 3'-flanking region are oH4-4 (5'-GCT AAC ATC ATA CCC TCC TG-3') (SEQ ID NO:37) and oH4-6 (5'-GTC GAC GTT AAC AGA AGC ATT GGA ATA CGC AT-3') (SEQ ID NO:38). The H4L 3'-flanking region as 0.5 kb SalI-SpeI fragment from pCR-H4L-fr was ligated between the sites SalI and XbaI right downstream to the H4L 5'-flanking region in pCR-D6R-fl, yielding the plasmid pCR-H4L-flanks.

In order to facilitate further cloning procedures, a synthetic linker containing the rare restriction sites SmaI, NotI, SfiI and RsrII was ligated between the H4L-flanking regions in pCR-H4L-flanks. The oligonucleotides used for the construction of the linker are oA8-11 and oA8-12. The plasmid pCR-H4L-flanks was thereafter linearized with MunI and HpaI (both sites are introduced by the PCR primers oH4-5 and oH4-6, respectively). The resulting plasmid designated pdH4L was ligated with a 3.9 kb SmaI double gene cassette from the plasmid pLGb described in Example 1 to yield pCR-H4Lf-ZG. In this construct the H4L orf is deleted completely in order to prevent rescue of the defect virus by the complementing cell line due to homologous recombination.

The construction of the d-H4L-ZG virus by in vivo recombination and plaque purification is done according to the scheme described in Example 1.

### Example 7: Construction of a complementing cell line with heatshock-inducible I7L function

The effect of the vaccinia host protein shut-off varies from cell type to cell type, which influences the effectiveness of complementation and thus determines the amount of plaque purifications necessary to obtain pure stocks of defective virus and the titers obtainable with the host cell lines. In order to make complementation more efficient, the essential open reading frame coding for the complementing function is cloned downstream of the promoter of the 70 kd human heatshock protein (Hsp70) gene (Hunt & Morimoto, supra). Transcription of the Hsp70-gene is induced by vaccinia infection and Hsp70 has been proposed to be involved in the assembly of the vaccinia virion (Jindal and Young, supra).

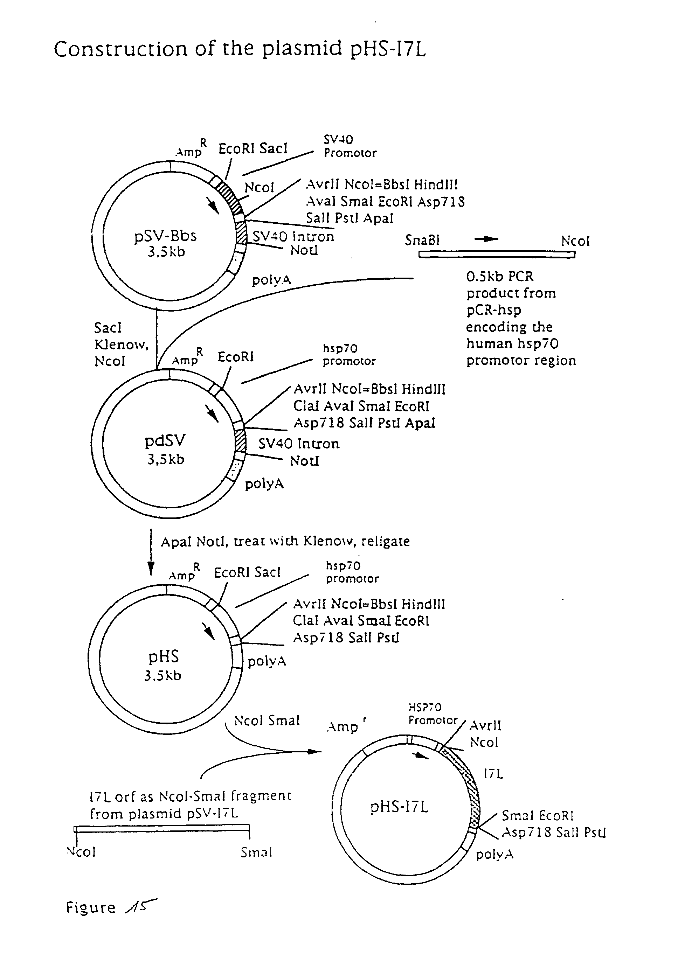

The first step is cloning of the Hsp70 promoter by PCR techniques. The oligonucleotides oHS-1, 5'- TACGTATGGA GACCAACACC CTTCC-3' (SEQ ID NO:39) and oHS-2, 5'- CCATGGATAT CGGGTTCCCT GCTCTCTGTC GG-3' (SEQ ID NO:40) were used to together with human DNA (Clontech, Inc.) as a template to obtain the human Hsp70 promoter sequences. The PCR product is cloned into the pCRII vector (InVitrogen, Inc.) yielding the plasmid pCR-Hsp. The SnaBI-NcoI promoter fragment is used to substitute the SV40 promoter in the plasmid pSV-Bbs (see Example 1) resulting, after removal of the SV40 intron present in this plasmid, in the plasmid pHS. This vector is used to insert open reading frames such as I7L, by ligating the NcoI-SmaI fragment of pSV-I7L (Example 1) with pHS yielding the plasmid pHS-I7L. See Figure 15.

To construct the complementing cell line, the co-transformation procedure (Wigler, supra) is performed. A suitable selection marker, such as the neomycin resistance gene present on the plasmid pSV2-neo (Southern, P.J. and Berg, P., J. Mol. Appl. Genet. 1: 327 (1982)) is used together with the plasmid pHS-I7L in a transfection experiment into Vero cells. The cells are transfected according to Graham and van der Eb, supra, and incubated in selective medium including the antibiotic G418 (DMEM, 10% fetal bovine serum, 500 µg/ml G-418). After 10-14 days, colonies become visible. These colonies are expanded, subcloned twice and then characterized further. The cell lines are further characterized by PCR analysis for the presence of the complementing gene construct. Western blots are used to show that the gene of interest, the I7L-protein is expressed upon induction. The final complementing cell line is named VHsp-I7L. The cell line is characterized as described in Example 1. Construction of defective virus d-I7L-ZG/Hsp is done as described in Example 1 for the virus d-I7L-ZG except that the cell line VHsp-I7L is used for complementation and that only five rounds of plaque purification are done to obtain the defective virus.

## Claims

1. A defective poxvirus that lacks a function imparted by an essential region of its parental poxvirus, the essential region being a region of the poxvirus genome that is needed for viability and infectivity of the parental poxvirus, said defective poxvirus comprising a foreign polynucleotide under transcriptional control of a poxvixus promoter.

2. A defective poxvirus according to claim 1, wherein said foreign polynucleotide is inserted into said essential region.

3. A defective poxvirus according to claim 2, wherein said essential region has been deleted from said defective poxvirus.

4. A defective poxvirus according to claim 3, wherein a marker replaces said essential region.

5. A defective poxvirus according to claim 4, wherein said foreign polynucleotide is inserted into said marker.

6. A defective poxvirus according to any of the preceding claims 1 to 5, wherein said parental poxvirus is vaccinia.

7. A defective poxvirus according to any of the preceding claims 1 to 6, wherein said essential region is an open reading frame selected from the group consisting of I7L, F18R, D13L, D6R, A8L, J1R, J3R, H4L, A10L and A3L.

8. A defective poxvirus according to any of the preceding claims 1 to 6, wherein said essential region is as open reading frame encoding the 14 kilodalton envelope protein or the 25 kilodalton structural protein.

9. A method of producing a protein, comprising the step of providing a defective poxvirus that lacks a function imparted by an essential region of its parental poxvirus, the essential region being a region of the poxvirus genome that is needed for viability and infectivity of the parental poxvirus, said defective poxvirus comprising a foreign polynucleotide under transcriptional control of a poxvirus promoter.

10. The method according to claim 9, wherein said providing step is accomplished by inserting said foreign polynucleotide into said essential region of said parental poxvirus.

11. A method according to claim 9, wherein said providing step is accomplished by replacing said essential region of said parental poxvirus with said foreign polynucleotide.

12. A method according to claim 9, wherein said providing step is accomplished by replacing said essential region of said parental poxvirus with a marker.

13. A method according to claim 12, wherein said polynucleotide is inserted into said marker.

14. A method according to claim 12, wherein said polynucleotide replaces said marker.

15. A method according to claim to any of the preceding claims 12 to 14, wherein said marker is gpt.

16. A vaccine comprising a defective poxvirus that lacks a function imparted by an essential region of its parental poxvirus, the essential region being a region of the poxvirus genome that is needed for viability and infectivity of the parental poxvirus, said defective poxvirus comprising a foreign polynucleotide under transcriptional control of a poxvirus promoter.

17. A vaccine according to claim 16, wherein said parental poxvirus is vaccinia.

18. A method of producing a vaccine, comprising the step of providing a defective poxvirus that lacks a function imparted by an essential region of its parental poxvirus, the essential region being a region of the poxvirus genome that is needed for viability and infectivity of the parental poxvirus, said defective poxvirus comprising a foreign polynucleotide under transcriptional control of a poxvirus promoter.

19. A method of producing a vaccine according to claim 16-17, wherein said parental poxvirus is vaccinia.

20. The use of a defective poxvirus according to any of the claims 1 to 8 for the preparation of a vaccine.

## Patentansprüche

1. Defekter Pockenvirus, dem eine Eigenschaft fehlt, die ihm durch einen wesentlichen Bereich seines Elternpockenvirus verliehen wird, wobei der wesentliche Bereich ein Bereich des Pockenvirusgenoms ist, der für die Lebensfähigkeit und Infektiosität des Elternpockenvirus erforderlich ist, wobei der defekte Pockenvirus ein fremdes Polynukleotid umfaßt, das unter der transkriptionalen Kontrolle eines Pockenviruspromoters steht.

2. Defekter Pockenvirus nach Anspruch 1, wobei das fremde Polynukleotid in den wesentlichen Bereich eingeführt wird.

3. Defekter Pockenvirus nach Anspruch 2, wobei der wesentliche Bereich aus dem defekten Pockenvirus deletiert wurde.

4. Defekter Pockenvirus nach Anspruch 3, wobei ein Marker den wesentlichen Bereich ersetzt.

5. Defekter Pockenvirus nach Anspruch 4, wobei das fremde Polynukleotid in diesen Marker eingeführt wird.

6. Defekter Pockenvirus nach einem der vorhergehenden Ansprüche 1 bis 5, wobei der Elternpockenvirus Vaccina ist.

7. Defekter Pockenvirus nach einem der vorhergehenden Ansprüche 1 bis 6, wobei der wesentliche Bereich ein offener Leserahmen, ausgewählt aus der Gruppe bestehend aus I7L, F18R, D13L, D6R, A8L, J1R, J3R, H4L, A10L und A3L, ist.

8. Defekter Pockenvirus nach einem der vorhergehenden Ansprüche 1 bis 6, wobei der wesentliche Bereich ein offener Leserahmen ist, der das 14 kD Hüllprotein oder das 25 kD Strukturprotein kodiert.

9. Verfahren zur Herstellung eines Proteins, umfassend den Schritt des Bereitstellens eines defekten Pockenvirus, dem eine Eigenschaft fehlt, die ihm durch einen wesentlichen Bereich seines Elternpockenvirus verliehen wird, wobei der wesentliche Bereich ein Bereich des Pockenvirusgenoms ist, der für die Lebensfähigkeit und Infektiosität des Elternpockenvirus erforderlich ist, wobei der defekte Pockenvirus ein fremdes Polynukleotid umfaßt, das unter der transkriptionalen Kontrolle eines Pockenviruspromoters steht.

10. Verfahren nach Anspruch 9, wobei der Schritt des Bereitstellens durch Einführen des fremden Polynukleotids in den wesentlichen Bereich des Elternpockenvirus vervollständigt wird.

11. Verfahren nach Anspruch 9, wobei der Schritt des Bereitstellens durch Austauschen des wesentlichen Bereichs des Elternpockenvirus mit dem fremden Polynukleotid vervollständigt wird.

12. Verfahren nach Anspruch 9, wobei der Schritt des Bereitstellens durch Austauschen des wesentlichen Bereichs des Elternpockenvirus mit einem Marker vervollständigt wird.

13. Verfahren nach Anspruch 12, wobei das Polynukleotid in den Marker eingeführt wird.

14. Verfahren nach Anspruch 12, wobei das Polynukleotid den Marker ersetzt.

15. Verfahren nach einem der vorhergehenden Ansprüche 12 bis 14, wobei der Marker gpt ist.

16. Impfstoff, umfassend einen defekten Pockenvirus, dem eine Eigenschaft fehlt, die ihm durch einen wesentlichen Bereich seines Elternpockenvirus verliehen wird, wobei der wesentliche Bereich ein Bereich des Pockenvirusgenoms ist, der für die Lebensfähigkeit und Infektiosität des Elternpockenvirus erforderlich ist, wobei der defekte Pockenvirus ein fremdes Polynukleotid umfaßt, das unter der transkriptionalen Kontrolle eines Pockenviruspromoters steht.

17. Impfstoff nach Anspruch 16, wobei der Elternpockenvirus Vaccina ist.

18. Verfahren zur Herstellung eines Impfstoffs, umfassend den Schritt des Bereitstellens eines defekten Pockenvirus, dem eine Eigenschaft fehlt, die ihm durch einen wesentlichen Bereich seines Elternpockenvirus verliehen wird, wobei der wesentliche Bereich ein Bereich des Pockenvirusgenoms ist, der für die Lebensfähigkeit und Infektiosität des Elternpockenvirus erforderlich ist, wobei der defekte Pockenvirus ein fremdes Polynukleotid umfaßt, das unter der transkriptionalen Kontrolle eines Pockenviruspromoters steht.

19. Verfahren zur Herstellung eines Impfstoffs gemäß Ansprüchen 16 und 17, wobei der Elternpockenvirus Vaccina ist.

20. Verwendung eines defekten Pockenvirus nach einem der Ansprüche 1 bis 8 zur Herstellung eines Impfstoffs.

## Revendications

1. Poxvirus défectif qui manque d'une fonction communiquée par une région essentielle de son poxvirus parental, la région essentielle étant une région du génome de poxvirus qui est nécessaire pour la viabilité et le pouvoir infectant du poxvirus parental, ledit poxvirus défectif comprenant un polynucléotide étranger sous contrôle transcriptionnel d'un promoteur de poxvirus.

2. Poxvirus défectif selon la revendication 1, dans lequel ledit polynucléotide étranger est inséré dans ladite région essentielle.

3. Poxvirus défectif selon la revendication 2, dans lequel ladite région essentielle a été délétée dudit poxvirus défectif.

4. Poxvirus défectif selon la revendication 3, dans lequel un marqueur remplace ladite région essentielle.

5. Poxvirus défectif selon la revendication 4, dans lequel ledit polynucléotide étranger est inséré dans ledit marqueur.

6. Poxvirus défectif selon l'une quelconque des revendications 1 à 5 précédentes, dans lequel ledit poxvirus parental est une vaccine.

7. Poxvirus défectif selon l'une quelconque des revendications 1 à 6 précédentes, dans lequel ladite région essentielle est un cadre de lecture ouvert choisi dans le groupe constitué par I7L, F18R, D13L, D6R, A8L, J1R, J3R, H4L, A10L et A3L.

8. Poxvirus défectif selon l'une quelconque des revendications 1 à 6 précédentes, dans lequel ladite région essentielle est un cadre de lecture ouvert codant pour la protéine 14 kilodaltons de l'enveloppe ou la protéine 25 kilodaltons de structure.

9. Procédé de production d'une protéine, comprenant l'étape de fournir un poxvirus défectif qui manque d'une fonction communiquée par une région essentielle de son poxvirus parental, la région essentielle étant une région du génome de poxvirus qui est nécessaire pour la viabilité et le pouvoir infectant du poxvirus parental, ledit poxvirus défectif comprenant un polynucléotide étranger sous contrôle transcriptionnel d'un promoteur de poxvirus.

10. Procédé selon la revendication 9, dans lequel ladite étape de fournir est accomplie en insérant ledit polynucléotide étranger dans ladite région essentielle dudit poxvirus parental.

11. Procédé selon la revendication 9, dans lequel ladite étape de fournir est accomplie en remplaçant ladite région essentielle dudit poxvirus parental avec ledit polynucléotide étranger.

12. Procédé selon la revendication 9, dans lequel ladite étape de fournir est accomplie en remplaçant ladite région essentielle dudit poxvirus parental avec un marqueur.

13. Procédé selon la revendication 12, dans lequel ledit polynucléotide est inséré dans ledit marqueur.

14. Procédé selon la revendication 12, dans lequel ledit polynucléotide remplace ledit marqueur.

15. Procédé selon l'une quelconque des revendications 12 à 14 précédentes, dans lequel ledit marqueur est gpt.

16. Vaccin comprenant un poxvirus défectif qui manque d'une fonction communiquée par une région essentielle de son poxvirus parental, la région essentielle étant une région du génome de poxvirus qui est nécessaire pour la viabilité et le pouvoir infectant du poxvirus parental, ledit poxvirus défectif comprenant un polynucléotide étranger sous contrôle transcriptionnel d'un promoteur de poxvirus.

17. Vaccin selon la revendication 16, dans lequel ledit poxvirus parental est une vaccine.

18. Procédé de production d'un vaccin, comprenant l'étape de fournir un poxvirus défectif qui manque d'une fonction communiquée par une région essentielle de son poxvirus parental, la région essentielle étant une région du génome de poxvirus qui est nécessaire pour la viabilité et le pouvoir infectant du poxvirus parental, ledit poxvirus défectif comprenant un polynucléotide étranger sous contrôle transcriptionnel d'un promoteur de poxvirus.

19. Procédé de production d'un vaccin selon les revendications 16-17, dans lequel ledit poxvirus parental est une vaccine.

20. Utilisation d'un poxvirus défectif selon l'une quelconque des revendications 1 à 8 pour la préparation d'un vaccin.
